# EUROPÄISCHE PATENTSCHRIFT

(11) **EP 0 519 291 B1**
(45) Veröffentlichungstag und Bekanntmachung des Hinweises auf die Patenterteilung: **08.01.1997**
(21) Anmeldenummer: 92109625.1
(22) Anmeldetag: 09.06.1992
(51) Int. Cl.: C07D 491/04, C07D 471/10, C07D 495/04, A61K 31/44

(54) **Aminomethyl-substituierte 2,3-Dihydropyrano(2,3-b)pyridine, Verfahren zu ihrer Herstellung und ihre Verwendung in Arzneimitteln**
Amino-methyl substituted 2,3-Dihydropyrano (2,3-b) pyridines, process for their preparation and their use in medicines
2,3-Dihydropyrano (2,3-b) pyridines amino-méthyl-substituées, procédé pour leur préparation et leur utilisation en tant que médicaments

(30) Priorität: 20.06.1991 DE 4120322
(43) Veröffentlichungstag der Anmeldung: 23.12.1992
(73) Patentinhaber: BAYER AG, 51368 Leverkusen (DE)
(72) Erfinder: Arlt, Dieter, Prof. Dr., W-5000 Köln 80 (DE); Heine, Hans-Georg, Dr., W-4150 Krefeld (DE); Schohe-Loop, Rudolf, Dr., W-5600 Wuppertal 1 (DE); Glaser, Thomas, Dr., W-5063 Overath 3 (DE); De Vry, Jean Marie Viktor, Dr., W-5064 Rösrath (DE)

(56) Entgegenhaltungen:
- EP-A- 0 199 400
- EP-A- 0 236 930
- EP-A- 0 270 947
- EP-A- 0 352 613
- EP-A- 0 433 149
- CHEMICAL SOCIETY REVIEWS, Band 18, 1979, Seiten 563- 580; C.W. THORNBER: "Isosterism and Molecular Modification in Drug Design"
- CHEMICAL ABSTRACTS, Band 96, Nr. 25, 21. Juni 1982, Seite 740, Zusammenfassung Nr. 217799j, Columbus, Ohio, US; R. PRATAP et al.: "Phenoxyalkylamines in semirigid conformation: synthesis and pharmacological activity of N1-(4'-chromanyl)-N4-arylpiperazines and N1-(2'- chromanylmethyl)-N4-arylpiperazines" & Indian J. Chem., Sect. B 1981, Band 20B, Nr. 12, Seiten 1063-1067

## Beschreibung

Die Erfindung betrifft Aminomethyl-substituierte 2,3-Dihydropyrano[2,3-b]pyridine, Verfahren zu ihrer Herstellung sowie ihre Verwendung in Arzneimitteln, insbesondere bei Erkrankungen des zentralen Nervensystems.

Es ist bereits bekannt, daß N-substituierte Aminomethyl-tetralin-Derivate und ihre heterocyclischen Analoga eine hohe Affinität zu Rezeptoren vom 5-HT₁-Typ besitzen und sowohl Wirkung auf das kardiovaskuläre als auch auf das zentralnervöse System zeigen [vgl. EP 352 613 A2].

Außerdem sind 2H-Pyrano[2,3-b]pyridin-4-ol-derivate mit antihypertensiver Wirkung und die 4-Carbonsäure-Derivate als Aldose Reductase Inhibitoren beschrieben [vgl. J. Med. Chem. 33 (11), 3023 - 3027; 33 (7), 1859 - 1865].

Die Erfindung betrifft Aminomethyl-substituierte 2,3-Dihydro-pyrano[2,3-b]pyridine der allgemeinen Formel (I) 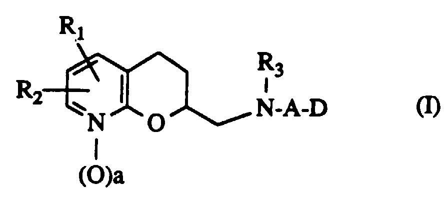 in welcher
- R¹ und R²: gleich oder verschieden sind und
für Wasserstoff, Halogen, Nitro, Trifluormethyl, Hydroxy oder für geradkettiges oder verzweigtes Alkyl oder Alkoxy mit jeweils bis zu 8 Kohlenstoffalomen stehen,
- a: für die Zahl 0 oder 1 steht,
- A: für geradkettiges oder verzweigtes Alkylen oder Alkenylen mit jeweils bis zu 8 Kohlenstoffatomen steht,
- D: für Cycloalkyl mit 3 bis 8 Kohlenstoffatomen oder für Aryl mit 6 bis 10 Kohlenstoffatomen steht, oder
für eine Gruppe der Formel -NR⁴R⁵, -OR⁶, -CO₂R⁷, -CO-NR⁸R⁹ steht,
worin
R⁴ und R⁵ gleich oder verschieden sind und Wasserstoff, Cycloalkyl mit 3 bis 8 Kohlenstoffatomen, Benzyl, geradkettiges oder verzweigtes Alkyl mit bis zu 8 Kohlenstoffatomen oder eine Gruppe der Formel -CO-R¹⁰ oder -SO₂R¹¹ bedeuten,
worin
R¹⁰ und R¹¹ gleich oder verschieden sind und geradkettiges oder verzweigtes Alkyl mit bis zu 8 Kohlenstoffatomen bedeuten oder Benzyl, Aryl mit 6 bis 10 Kohlenstoffatomen oder einen 5- bis 7-gliedrigen ungesättigten Heterocyclus mit bis zu 4 Heteroatomen aus der Reihe S, N oder O bedeuten, die gegebenenfalls durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind, oder
- R⁴ und R⁵: gemeinsam mit dem Stickstoffatom einen Rest der Formel 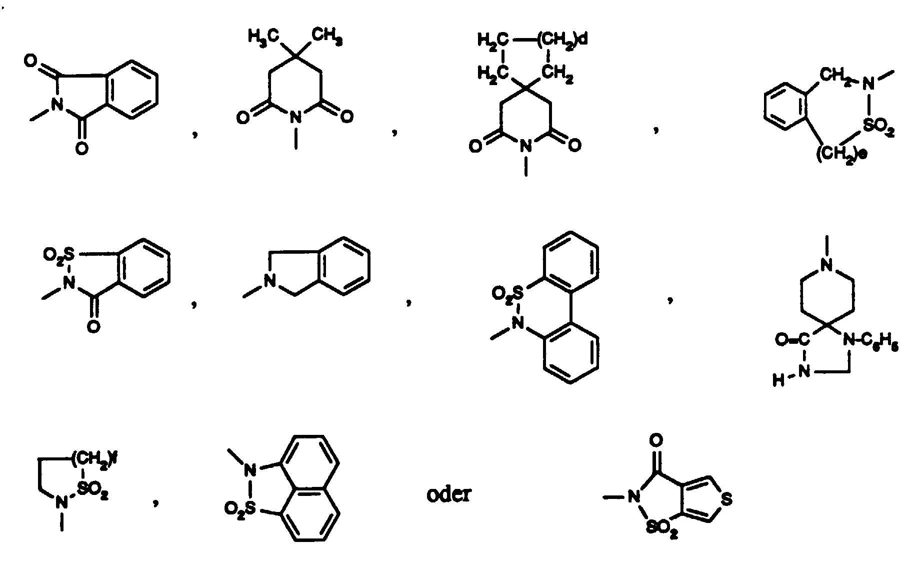 bilden,
worin
d und f gleich oder verschieden sind und die Zahl 1 oder 2 bedeuten,
e die Zahl 0, 1 oder 2 bedeutet,
- R⁶: Wasserstoff, Cycloalkyl mit 3 bis 8 Kohlenstoffatomen, geradkettiges oder verzweigtes Alkyl, Alkylcarbonyl oder Carbamoyl mit jeweils bis zu 6 Kohlenstoffatomen bedeutet, oder
Benzyl, Aryl mit 6 bis 10 Kohlenstoffatomen oder einen 5-gliedrigen ungesättigten Heterocylus mit bis zu 4 Heteroatomen aus der Reihe N, S oder O bedeutet, die gegebenenfalls
durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
- R⁷: Wasserstoff, geradkettiges oder verzweigtes Alkyl mit bis zu 6 Kohlenstoffatomen oder Benzyl bedeutet,
- R⁸ und R⁹: gleich oder verschieden sind und Wasserstoff, geradkettiges oder verzweigtes Alkyl mit bis zu 8 Kohlenstoffatomen, Benzyl oder Aryl mit 6 bis 10 Kohlenstoffatomen bedeuten,
- R³: für Wasserstoff, geradkettiges oder verzweigtes Alkyl mit bis zu 8 Kohlenstoffatomen oder Benzyl steht, oder
für die Gruppe -A-D steht,
worin
A und D die oben angegebene Bedeutung haben,
und deren Salze.

Die erfindungsgemäßen Verbindungen können auch in Form ihrer Salze vorliegen. Im allgemeinen seien hier Salze mit anorganischen oder organischen Säuren genannt. Bevorzugt sind physiologisch unbedenkliche Salze.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Physiologisch unbedenkliche Salze der aminomethyl-substituierten 2,3-Dihydropyrano[2,3-b]pyridine können Salze der erfindungsgemäßen Stoffe mit Mineralsäuren, Carbonsäuren oder Sulfonsäuren sein. Besonders bevorzugt sind beispielsweise Salze mit Chlorwasserstoffsäure, Methansulfonsäure, Ethansulfonsäure, Toluolsulfonsäure, Benzolsulfonsäure, Naphthalindisulfonsäure, Essigsäure, Propionsäure, Milchsäure, Oxalsäure, Malonsäure, Bernsteinsäure, Maleinsäure, Fumarsäure, Äpfelsäure, Weinsäure, Zitronensäure oder Benzoesäure.

Heterocyclus im Rahmen der oben angegebenen Definition steht im allgemeinen für einen 5- bis 7-gliedrigen aromatischen Ring, der als Heteroatome Sauerstoff, Schwefel und/oder Stickstoff enthalten kann und an den ein weiterer aromatischer Ring ankondensiert sein kann. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyridyl, Pyrimidyl, Pyrazinyl, Pyridazinyl, Chinolyl, Isochinolyl, Chinoxazolyl, Chinolyl, Thiazolyl, Benzothiazolyl, Isothiazolyl, Oxazolyl, Isoxazolyl, Imidazolyl, Benzimidazolyl, Pyrazolyl und Indolyl.

Bevorzugt sind Verbindungen der allgemeinen Formel (I),
in welcher
- R¹ und R²: gleich oder verschieden sind und
für Wasserstoff, Fluor, Chlor, Brom, Nitro, Trifluormethyl, Hydroxy oder für geradkettiges oder verzweigtes Alkyl oder Alkoxy mit jeweils bis zu 6 Kohlenstoffatomen stehen
- a: für die Zahl 0 oder 1 steht,
- A: für geradkettiges oder verzweigtes Alkylen oder Alkenylen mit jeweils bis zu 6 Kohlenstoffatomen steht,
- D: für Cyclopropyl, Cyclopentyl, Cyclohexyl oder Phenyl steht, oder für eine Gruppe der Formel -NR⁴R⁵, -OR⁶, -CO₂R⁷, -CO-NR⁸R⁹ steht,
worin
R⁴ und R⁵ gleich oder verschieden sind und Wasserstoff, Cyclopropyl, Cyclopentyl, Cyclohexyl, Benzyl, geradkettiges oder verzweigtes Alkyl mit bis zu 6 Kohlenstoffatomen oder eine Gruppe der Formel -CO-R¹⁰ oder -SO₂R¹¹ bedeuten,
worin
R¹⁰ und R¹¹ gleich oder verschieden sind und geradkettiges oder verzweigtes Alkyl mit bis zu 6 Kohlenstoffatomen bedeuten, oder Benzyl oder Phenyl bedeuten, die gegebenenfalls durch Nitro, Cyano, Trifluormethyl, Fluor, Chlor, Carboxy, Hydroxy oder durch geradkettiges oder verzweigtes Alkyl, Alkoxy oder Alkoxycarbonyl mit jeweils bis zu 4 Kohlenstoffatomen substituiert sind oder
R⁴ und R⁵ gemeinsam mit dem Stickstoffatom einen Rest der Formel 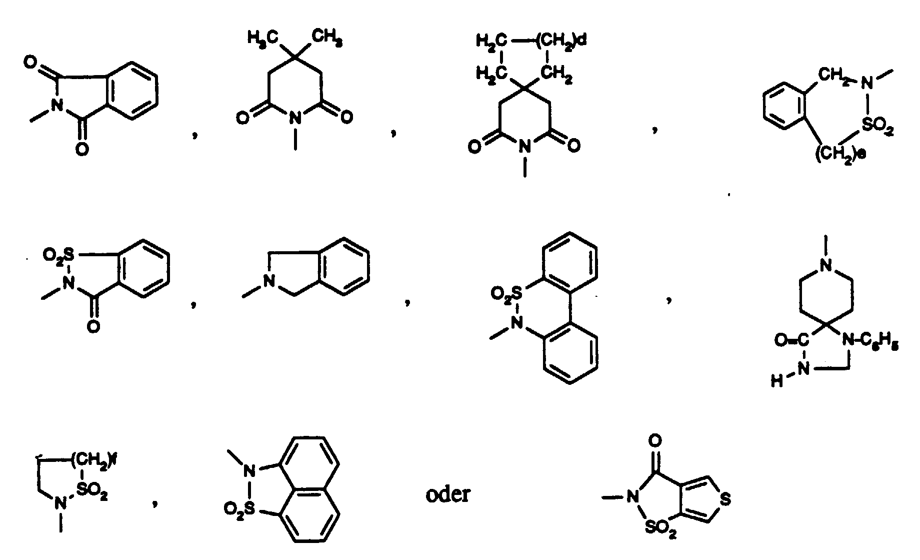 bilden,
worin
d und f gleich oder verschieden sind und die Zahl 1 oder 2 bedeuten,
e die Zahl 0, 1 oder 2 bedeutet,
R⁶ Wasserstoff, geradkettiges oder verzweigtes Alkyl, Alkylcarbonyl oder Carbamoyl mit jeweils bis zu 4 Kohlenstoffatomen bedeutet oder Benzyl oder Phenyl bedeutet, die gegebenenfalls durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
R⁷ Wasserstoff, geradkettiges oder verzweigtes Alkyl mit bis zu 4 Kohlenstoffatomen oder Benzyl bedeutet,
R⁸ und R⁹ gleich oder verschieden sind und Wasserstoff, geradkettiges oder verzweigtes Alkyl mit bis zu 6 Kohlenstoffatomen, Benzyl oder Phenyl bedeuten,
- R³: für Wasserstoff, geradkettiges oder verzweigtes Alkyl mit bis zu 6 Kohlenstoffatomen oder Benzyl steht,
oder
für die Gruppe -A-D steht,
worin
A und D die oben angegebene Bedeutung haben,
und deren Salze.

Besonders bevorzugt sind Verbindungen der allgemeinen Formel (I),
in welcher
- R¹ und R²: gleich oder verschieden sind und
für Wasserstoff, Fluor, Chlor, Trifluormethyl, Hydroxy oder für geradkettiges oder verzweigtes Alkyl oder Alkoxy mit jeweils bis zu 4 Kohlenstoffatomen stehen,
- a: für die Zahl 0 oder 1 steht,
- A: für geradkettiges oder verzweigtes Alkylen oder Alkenylen mit jeweils bis zu 4 Kohlenstoffatomen steht,
- D: für Cyclopropyl, Cyclopentyl, Cyclohexyl, Hydroxy oder Phenyl steht, oder für eine Gruppe der Formel -NR⁴R⁵ steht,
worin
R⁴ und R⁵ gleich oder verschieden sind und Wasserstoff, Cyclopropyl, Cyclopentyl, Cyclohexyl, Benzyl oder geradkettiges oder verzweigtes Alkyl mit bis zu 4 Kohlenstoffatomen bedeuten,
oder
R⁴ und R⁵ gemeinsam mit dem Stickstoffatom einen Rest der Formel 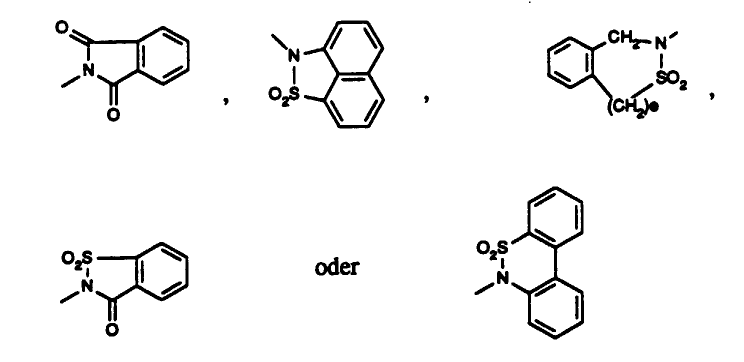 bilden,
worin
e die Zahl 0 oder 1 bedeutet,
- R³: für Wasserstoff, geradkettiges oder verzweigtes Alkyl mit bis zu 4 Kohlenstoffatomen oder Benzyl steht,
oder
für die Gruppe -A-D steht,
worin
A und D die oben angegebene Bedeutung haben,
und deren Salze.

Außerdem wurde ein Verfahren zur Herstellung der erfindungsgemäßen Verbindungen der allgemeinen Formel (I) gefunden, dadurch gekennzeichnet, daß man
Amine der allgemeinen Formel (II) 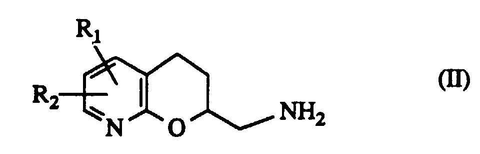 in welcher
R¹ und R² die oben angegebene Bedeutung haben,
mit Verbindungen der allgemeinen Formel (III)

L-A-D (III)

in welcher
A und D die oben angegebene Bedeutung haben
und
- L: für eine typische Abgangsgruppe, wie beispielsweise Brom, Chlor, Jod, Tosyl oder Mesyl, vorzugsweise für Brom steht,

in inerten Lösemitteln, gegebenenfalls in Anwesenheit einer Base und eines Reaktionsbeschleunigers umsetzt,
und im Fall, daß R³ nicht für Wasserstoff steht, eine Alkylierung,
und im Fall der 2,3-Dihydropyrano[2,3-b]pyridin-N-oxide (a = 1) eine Oxidation nach üblichen Methoden anschließt.

Als Lösemittel können Wasser oder die üblichen organischen Lösemittel verwendet werden, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden. Bevorzugt ist Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin, oder N-Methylpiperidin, oder Amide wie Natriumamid oder Lithiumdiisopropylamid, oder metallorganische Verbindungen wie Butyllithium oder Phenyllithium. Bevorzugt ist Triethylamin.

Die Base wird hierbei in einer Menge von 1 bis 5, bevorzugt von 1 bis 2 Mol, bezogen auf 1 Mol der Verbindungen der allgemeinen Formel (III) eingesetzt. Die Verbindungen der allgemeinen Formel (III) werden bevorzugt in einer bis zur 3-fachen, bevorzugt in einer bis zur 1,5-fachen Überschußmenge über die Verbindungen der Formel (II) eingesetzt.

Als Reaktionsbeschleuniger werden im allgemeinen Alkalijodide, bevorzugt Natrium- oder Kaliumjodid, in einer Menge von 0,01 mol bis 0,5 mol, bevorzugt von 0,01 mol bis 0,1 mol bezogen auf 1 mol der Verbindungen der allgemeinen Formel (III) eingesetzt.

Die Umsetzung wird im allgemeinen in einem Temperaturbereich von 0°C bis +150°C, bevorzugt in einem Bereich von Raumtemperatur bis +80°C, durchgeführt.

Die Reaktionsmischung wird im allgemeinen bei Normaldruck durchgeführt. Es ist jedoch ebenso möglich, die Reaktion bei erhöhtem oder erniedrigtem Druck (z.B. 0,5 bis 3 bar) durchzuführen.

Die Alkylierung erfolgt im allgemeinen in einem der oben aufgeführten Lösemitteln, vorzugsweise in Dimethylformamid, in einem Temperaturbereich von 0°C bis +158°C, vorzugsweise von Raumtemperatur bis +100°C.

Als Alkylierungsmittel bei dem Verfahren können beispielsweise (C₁-C₈)-Alkylhalogenide, Sulfonsäureester oder substituierte oder unsubstituierte (C₁-C₆)-Dialkyl- oder (C₆-C₁₀)-Diarylsulfate, vorzugsweise Methyljodid, p-Toluolsulfonsäureester oder Dimethylsulfat eingesetzt werden.

Die Oxidation zum N-oxid erfolgt im allgemeinen in einem der oben aufgeführten Lösemittel, vorzugsweise in Methylenchlorid mit Oxidationsmitteln wie beispielsweise Metachlorperbenzoesäure, Wasserstoffperoxid oder Peressigsäure, vorzugsweise mit Metachlorperbenzoesäure in einem Temperaturbereich von 0°C bis 120°C, bevorzugt von 20°C bis 80°C.

Sowohl die Oxidation als auch die Alkylierung können im allgemeinen bei Normaldruck durchgeführt werden. Es ist jedoch ebenso möglich, die Reaktion bei erhöhtem oder erniedrigtem Druck (z.B. 0,5 bis 3 bar) durchzuführen.

Die Verbindungen der allgemeinen Formel (II) sind neu und können hergestellt werden, indem man
Verbindungen der allgemeinen Formel (IV) 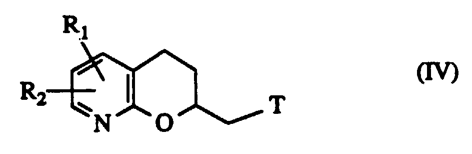 in welcher
R¹ und R² die oben angegebene Bedeutung haben,
und
- T: für Halogen, vorzugsweise für Brom und/oder Chlor steht,
gegebenenfalls als Gemisch (T-Br, T-Cl) zunächst mit Kaliumphthalimid in inerten Lösermitteln und unter Schutzgasatmosphäre zu den Verbindungen der allgemeinen Formel (V) 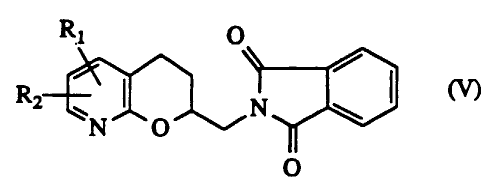 in welcher
R¹ und R² die oben angegebene Bedeutung haben,
umsetzt,
und in einem zweiten Schritt durch Umsetzung mit 2-Amino-ethanol oder Hydrazinhydrat die Aminfunktion freisetzt.

Als Lösemittel für die Umsetzung mit Kaliumphthalimid / 2-Aminoethanol eignen sich die oben angegebenen Lösemittel, vorzugsweise Dimethylformamid neben Toluol, wobei im zweiten Schritt 2-Amino-ethanol gegebenenfalls selbst durch Einsatz in großem Überschuß als Lösemittel fungiert

Die Reaktionen verlaufen in einem Temperaturbereich von +50°C bis +150°C, bevorzugt von +60°C bis +120°C und Normaldruck.

Die Verbindungen der allgemeinen Formel (III) sind bekannt [vgl. Beilstein 2, 197, 201, 250, 278; 3, 9, 10; 21, 401, 462, 463].

Die Verbindungen der allgemeinen Formel (V) sind neu und können nach dem oben aufgeführten Verfahren hergestellt werden.

Die Verbindungen der allgemeinen Formel (IV) sind ebenfalls neu und können hergestellt werden, indem man Verbindungen der allgemeinen Formel (VI) 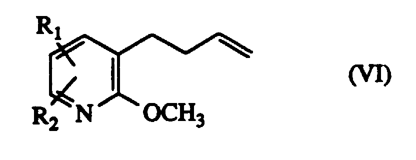 in welcher
R¹ und R² die oben angegebene Bedeutung haben,
zunächst durch Bromierung,vorzugsweise mit elementarem Brom, in einem der oben aufgeführten Lösemittel, vorzugsweise Methylenchlorid in die Verbindungen der allgemeinen Formel (VII) 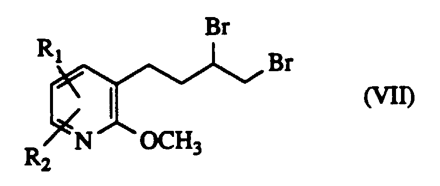 in welcher
R¹ und R² die oben angegebene Bedeutung haben,
überführt und anschließend in einem 2-Stufenprozeß durch Umsetzung mit verdünnter Salzsäure und Natriumcarbonat/Natriumhydrogencarbonat-Lösung zum 6-gliedrigen Heterocyclus ringschließt.

Die Verbindungen der allgemeinen Formel (VII) sind ebenfalls neu.

Die Verbindungen der allgemeinen Formel (VI) sind neu und können hergestellt werden, indem man Verbindungen der allgemeinen Formel (VIII) 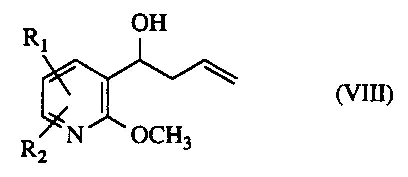 in welcher
R¹ und R² die oben angegebene Bedeutung haben,
durch Umsetzung mit den üblichen Chlorierungsmitteln, vorzugsweise Thionylchlorid in einem der oben aufgeführten Lösemitteln, vorzugsweise Dichlormethan chloriert und in einem Folgeschritt beispielsweise mit durch Kupfer aktiviertem Zink, in einem der oben aufgeführten Lösemitteln, vorzugsweise Methanol oder mit Tributylzinnhydrid in Kohlenwasserstoffen, in diesem Fall vorzugsweise mit Toluol, reduziert.

Die oben aufgeführten Reaktionen werden im allgemeinen in einem Temperaturbereich von 0°C bis +120°C, vorzugsweise von +20°C bis +100°C und Normaldruck durchgeführt.

Die Verbindungen der allgemeinen Formel (VIII) sind ebenfalls neu und können beispielsweise hergestellt werden, indem man entweder den literaturbekannten 2-Methoxypyridin-3-carbaldehyd (R¹/R² = H) oder dessen substituierte Derivate (R¹/R² ≠ H) mit Allylbromid und Aluminium in Anwesenheit von Quecksilber-II-chlorid in einem der oben angegebenen Ether, vorzugsweise Tetrahydrofuran, umsetzt.

Die Reaktion wird im allgemeinen in einem Temperaturbereich von -60°C bis +50°C, vorzugsweise von -60°C bis +25°C und Normaldruck durchgeführt.

Die erfindungsgemäßen Stoffe zeigen überraschenderweise eine vorteilhafte Wirkung auf das Zentralnervensystem und können zur therapeutischen Behandlung von Menschen und Tieren verwendet werden. Gegenüber den bereits bekannten strukturverwandten Verbindungen zeichnen sie sich durch eine größere Selektivität für den 5-HT_{1A}-Rezeptor durch zum Teil serotoninantagonistische Wirkung und geringere Nebenwirkungen aus.

Sie haben agonistische, partiell agonistische oder antagonistische Wirkungen am Serotonin-Rezeptor. Im Vergleich zu den strukturverwandten bekannten Verbindungen weisen sie überraschenderweise eine größere therapeutische Breite auf.

Die in der vorliegenden Erfindung beschriebenen hochaffinen Liganden für den Serotonin-1-Rezeptor stellen somit Wirkstoffe zur Bekämpfung von Krankheiten dar, die durch Störungen des serotoninergen Systems, insbesondere bei Involvierung von Rezeptoren, die hohe Affinität zum 5-Hydroxytryptamin (Serotonin) besitzen (5-HT₁-Typ), gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlaf- und Nahrungsaufnahmestörungen. Weiterhin sind sie geeignet zur Beseitigung kognitiver Defizite, zur Verbesserung von Lern- und Gedächtnisleistungen und zur Behandlung der Alzheimer'schen Krankheit.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar.

Auch eignen sie sich zur Prophylaxe und Bekämpfung der Folgen cerebraler Infarktgeschehen wie Schlaganfall, akutes Schädelhirn Trauma, cerebraler Ischämie sowie deren Folgeprozesse. Ebenso können die erfindungsgemässen Verbindungen zur Bekämpfung von Erkrankungen des Intestinaltraktes, die durch Störungen des serotoninergen Systems wie auch durch Störungen des Kohlenhydrathaushaltes gekennzeichnet sind.

### Affinität zum 5-HT₁-Rezeptor

In Tabelle 1 wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu ³H-Serotonin verwendet.

**Tabelle 1**

| Verbindung des Beispiels | Kᵢ (nmol/l) |
|---|---|
| 4 | 1,0 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

3-(1-Hydroxy-but-3-en-1-yl)-2-methoxypyridin

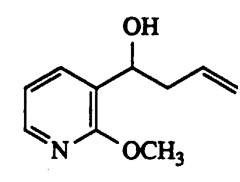

29,9 g (1,1 g-at) Aluminiumschuppen und 100 mg Quecksilber-2-chlorid werden in 300 ml trockenem Tetrahydrofuran unter Argon suspendiert. Man erwärmt auf 40°C und tropft langsam 1 - 2 ml von 214,0 g (1,77 mol) Allylbromid in 250 ml trockenem Tetrahydrofuran zu. Hierbei steigt die Temperatur auf etwa 50°C an. Man tropft nun die Allylbromid-Lösung unter Rühren so zu, daß die Temperatur der Lösung 50°C nicht überschreitet. Anschließend rührt man 1 Stunde bei 60°C nach, kühlt die Lösung auf -60°C ab und tropft bei dieser Temperatur 112,4 g (0,765 mol) 2-Methoxypyridin-3-carbaldehyd in 250 ml trockenem Tetrahydrofuran zu. Man rührt 1 Stunde bei 0°C und 2 Stunden bei 20°C nach. Anschließend tropft man unter Kühlen 500 ml gesättigte Ammoniumchlorid-Lösung zu, rührt 0,5 Stunden nach, filtriert und engt das Filtrat im Vakuum ein. Den Rückstand nimmt man in Essigsäureethylester auf, wäscht mit Wasser und trocknet über Natriumsulfat. Man erhält 136,1 g hellgelbes Öl, das fraktionierend destilliert wird.
Ausbeute: 110,9 g (81% der Theorie)
Sdp.: 91 - 100°C/0,5 Torr.

### Beispiel II

3-(1-Chlor-but-3-en-1-yl)-2-methoxypyridin

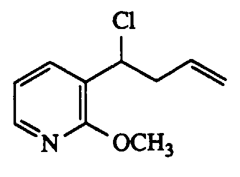

56,5 g (0,32 mol) der Verbindung aus Beispiel I werden in 400 ml trockenem Dichloromethan mit 277 ml (3,8 mol) Thionylchlorid über Nacht bei 20 - 30°C gerührt. Anschließend werden im Wasserstrahlvakuum bei 20 - 30°C Dichlormethan und nichtumgesetztes Thionylchlorid abgezogen. Der Rückstand wird zwischen Dichlormethan und wäßriger gesättigter Natriumbicarbonatlösung verteilt. Die organische Phase wird abgetrennt, getrocknet und im Vakuum eingedampft. 59,3 g rohe Titelverbindung als Öl werden erhalten.
Ausbeute: 94% der Theorie

### Beispiel III

3-(But-3-en-1-yl)-2-methoxypyridin

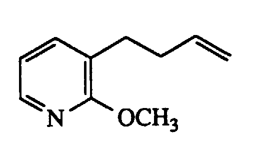

118,5 g (0,54 mol) der Verbindung aus Beispiel II werden in 1000 ml trockenem Methanol mit insgesamt 71,4 g Zink (mit Kupfer aktiviert) versetzt und 2 Stunden zum Rückfluß erhitzt. Der Niederschlag wird abfiltriert, das Filtrat anschließend im Vakuum eingedampft Fraktionierendes Destillieren des Rückstandes liefert 20,0 g der Titelverbindung vom Sdp. 96°C / 20 Torr.
Ausbeute: 23% der Theorie

### Beispiel IV

3-(3,4-Dibrom-but-1-yl)-2-methoxypyridin

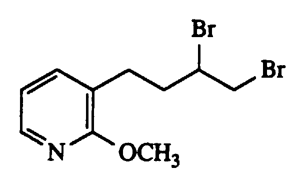

14,4 g (88 mmol) Brom in 30 ml trockenem Dichlormethan werden bei 20 - 30°C zu der Lösung von 14,0 g (88 mmol) der Verbindung aus Beispiel III in 70 ml trockenem Dichlormethan unter Rühren getropft Nach 15 Minuten versetzt man das Reaktionsgemisch mit kalter gesättigter Natriumbicarbonat-Lösung, fügt einige ml Natriumsulfit-Lösung hinzu und trennt die Phasen. Die Dichlormethanphase wird getrocknet und im Vakuum eingedampft. 28,1 g rohe Titelverbindung werden als Öl erhalten.

Ausbeute: 100% der Theorie

### Beispiel V und Beispiel VI

(2-Brom-methyl)-2,3-dihydropyrano(2,3-b)pyridin (Beispiel V) 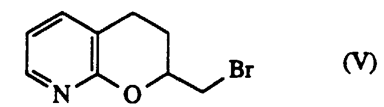 und (2-Chlor-methyl)-2,3-dihydropyrano(2,3-b)pyridin (Beispiel VI)

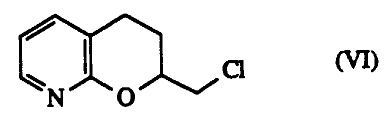

28,1 g (88 mmol) rohe Verbindung aus Beispiel IV werden in 212 ml 5%ige wäßrige Salzsäure unter Stickstoff und Rühren 4,5 Stunden zum Rückfluß erhitzt. Nach dem Abkühlen auf 20°C wird das Reaktionsgemisch mit n-Hexan extrahiert. Anschließend wird die wäßrige Phase durch Zugabe von Soda-Lösung alkalisch gestellt und mit Dichlormethan extrahiert. Eindampfen dieser Phase im Vakuum liefert 15,0 g kristallines Produkt, das die Titelverbindungen im Verhältnis 12:5 (V/VI) enthält.
Ausbeute: ca. 79% der Theorie

### Beispiel VII

2-Phthalimidomethyl-2,3-dihydrpyrano(2,3-b)pyridin

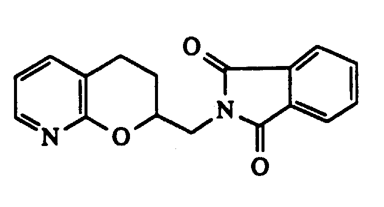

15,0 g (70 mmol) eines Gemisches der Verbindungen aus Beispiel V und VI (Verhältnis 12:5) werden mit 16,0 g (86 mmol) Kaliumphthalimid in 150 ml Dimethylformamid unter Stickstoff und Rühren 20 Stunden auf 100°C erhitzt. Dimethylformamid wird anschließend im Vakuum abdestilliert und der Rückstand zwischen Dichlormethan und Wasser verteilt. 24,0 g Kristalle vom Schmelzbereich 152-158°C werden erhalten.

NMR (CDCl₃): 1,70 - 2,00 (m, 1H); 2,00- 2,20 (m, 1H); 2,80 - 2,90 (m, 2H); 3,80 - 3,95 und 4,10 - 4,25 (AB-System, 2H); 4,50 - 4,65 (m, 1H); 6,75 - 6,90 (m, 1H); 7,30-7,40 (m, 1H); 7,65 - 7,80 (m, 2H); 7,80 - 7,95 (m, 2H) und 8,00 - 8,10 (m, 1H) ppm.
Ausbeute: ca. 100% der Theorie

### Beispiel VIII

2-Aminomethyl-2,3-dihydropyrano(2,3-b)-pyridin 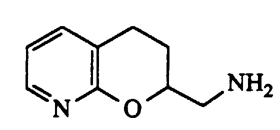 3,2 g (11 mmol) der Verbindung aus Beispiel VII und 9,2 g (150 mmol) 2-Aminoethanol werden unter Stickstoff auf 80°C erwärmt. Nach 5 Minuten kühlt man ab und verteilt das Reaktionsgemisch zwischen 70 ml Toluol und 92 ml 5%iger NaCl-Lösung. Man trennt die Phasen und extrahiert die wäßrige Phase mit Dichlormethan. Die vereinigten organischen Phasen werden getrocknet und im Vakuum eingedampft Man erhält 1,7 g der Titelverbindung als hellgelbes Öl.
Ausbeute: 94% der Theorie
NMR (CDCl₃): 1,73 - 1,85 (m, 1H); 1,93 - 2,00 (m, 3H, 2H tauschen auf Zusatz von D₂O); 2,74 2,95 (m) sowie 2,96 (quasi d, 4H); 4,09 - 4,18 (m, 1H); 6,81 - 6,86 (m, 1H); 7,35 - 7,37 (m, 1H) und 8,04 - 8,05 (m, 1H) ppm.

### Herstellungsbeispiele

### Beispiel 1 und Beispiel 2

2-N-Bis-(4-butylsaccharinyl)-aminomethyl-2,3-dihydropyrano[2,3-b]pyridin(1) 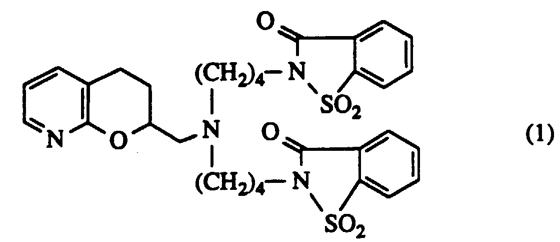 und
2-N-(4-butylsaccharinyl)-aminomethyl-2,3-dihydropyrano(2,3-b)pyridin (2)

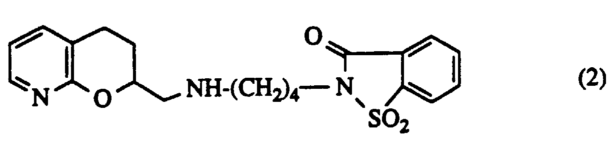

2,1 g (11 mmol) der Verbindung aus Beispiel VIII, 3,5 g (11 mmol) 4-Brombutylsaccharin, 1,1 g (11 mmol) Triethylamin und 10 mg Natriumjodid werden in 10 ml Dimethylformamid 9 Stunden bei 60°C (vollständiger Umsatz) unter Argon gerührt. Dimethylformamid wird bei 0,01 Torr und 40°C abdestilliert. Den Rückstand verteilt man zwischen Wasser und Dichlormethan und trennt die Phasen. Die organische Phase wird mit 0,1 n NaOH alkalisch gestellt, mit Wasser gewaschen, anschließend über Natriumsulfat getrocknet und eingedampft. 4,0 g hellgelbes Öl werden erhalten, die an 200 g Kieselgel mit Toluol/Essigsäureethylester (Gradient) chromatographiert werden. 0,7 g 2-N-Bis(4-butylsaccharinyl)-aminomethyl-2,3-dihydropyrano(2,3-b)pyridin (1) werden erhalten.
Ausbeute: 10% der Theorie
NMR (CDCl₃): 1,54 - 1,86 (m, 9H); 2,19 - 2,34 (m, 1H); 2,54 - 2,86 (m, 8H); 3,76 - 3,82 (m, 4H); 4,20 - 4,30 (m, 1H); 6,77 - 6,82 (m, 1H); 7,34 - 7,37 (m, 1H); 7,78 - 7,91 (m, 6H) und 8,01 - 8,04 (m, 3H) ppm.

Die wäßrige Phase (pH 5,5 ) wird mit 0,1 n NaOH alkalisch gestellt und mit Dichlormethan ausgeschüttelt. Die Dichlormethanextrakte werden anschließend mit Wasser gewaschen, über Natriumsulfat getrocknet und eingedampft. Den Rückstand (1,2 g) chromatographiert man an 60 g Kieselgel mit Toluol/Essigsäuremethylester 1:1 und erhält 0,2 g 2-N-(4-Butylsaccharinyl)-aminomethyl-2,3-dihydropyrano(2,3-b)pyridin (2).
Ausbeute: 5% der Theorie
NMR (CDCl₃): 1,62 - 2.05 (m, 6H); 2,74 - 3,40 (m, 7H); 3,76 - 3,82 (m, 2H); 4,32 - 4,00 (m, 1H); 6,80 - 6,85 (m, 1H); 7,36 - 7,39 (m, 1H); 7,79 - 7,93 (m, 3H) und 8,02 - 8,05 (m, 2H) ppm.

### Beispiel 3

2-N-Bis-(4-butylsaccharinyl)-aminomethyl-2,3-dihydropynno(2,3-b)pyridin-HCl-Salz 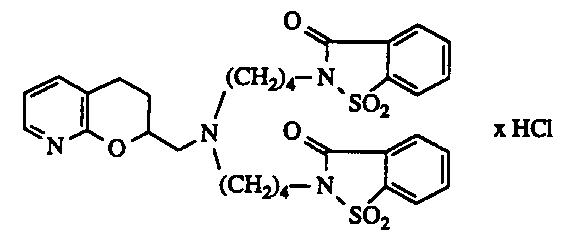 0,7 g der Verbindung aus Beispiel 1 werden in 100 ml Diethylether gelöst und mit 1 m etherischer Salzsäure schwach sauer (pH 4 - 5) gestellt. Man erhält 0,5 g der Titelverbindung als amorphes Salz.
C₃₁H₃₄N₄O₇S₂ x 2HCl
Ber.: C 52,32 H 5,10 N 7,87
Gef.: C 53,1 - 53,5 H 5,01 - 5,02 N 7,88 -7,97

### Beispiel 4

2-N-(4-Butylsaccharinyl)-aminomethyl-2,3 dihydropylano(2,3-b)pyridin-HCl-Salz 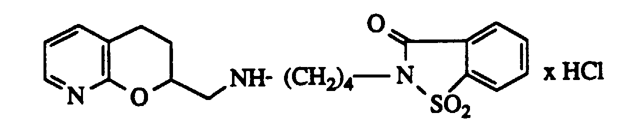 0,2 g der Verbindung aus Beispiel 2 werden in 50 ml Diethylether gelöst und mit 1 m etherischer Salzsäure schwach sauer (pH 4 - 5) gestellt. Der hygroskopische Niederschlag wird abgesaugt und mit Diethylether gewaschen. Man erhält 0,1 g kristallines Salz der Titelverbindung vom Schmelzbereich 42 - 50°C.
NMR (CDCl₃ / DMSO-d₆): charakterische Signale bei 4,6 - 4,8 (breites m, 2-3H); 7,0 - 7,1 (m, 1H), 7,7 - 7,8 (m, 1H); 7,9 - 8,1 (m, 4H); 8,2 - 8,3 (m, 1H) sowie 9,3 und 9,5 (m, jeweils 1H) ppm.

## Patentansprüche

1. Aminomethyl-substituierte 2,3-Dihydropyrano[2,3-b]pyridine der allgemeinen Formel 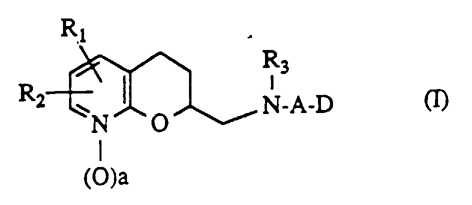 in welcher
R¹ und R² gleich oder verschieden sind und
für Wasserstoff, Halogen, Nitro, Trifluormethyl, Hydroxy oder für geradkettiges oder verzweigtes Alkyl oder Alkoxy mit jeweils bis zu 8 Kohlenstoffatomen stehen,
a für die Zahl 0 oder 1 steht,
A für geradkettiges oder verzweigtes Alkylen oder Alkenylen mit jeweils bis zu 8 Kohlenstoffatomen steht,
D für Cycloalkyl mit 3 bis 8 Kohlenstoffatomen oder für Aryl mit 6 bis 10 Kohlenstoffatomen steht, oder
für eine Gruppe der Formel -NR⁴R⁵, -OR⁶, -CO₂R⁷, -CO-NR⁸R⁹ steht,
worin
R⁴ und R⁵ gleich oder verschieden sind und Wasseistoff, Cycloalkyl mit 3 bis 8 Kohlenstoffatomen, Benzyl, geradkettiges oder verzweigtes Alkyl mit bis zu 8 Kohlenstoffatomen oder eine Gruppe der Formel -CO-R¹⁰ oder -SO₂R¹¹ bedeuten,
worin
R¹⁰ und R¹¹ gleich oder verschieden sind und geradkettiges oder verzweigtes Alkyl mit bis zu 8 Kohlenstoffatomen bedeuten oder Benzyl, Aryl mit 6 bis 10 Kohlenstoffatomen oder einen 5- bis 7-gliedrigen ungesättigten Heterocyclus mit bis zu 4 Heteroatomen aus der Reihe S, N oder O bedeuten, die gegebenenfalls durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
oder
R⁴ und R⁵ gemeinsam mit dem Stickstoffatom einen Rest der Formel 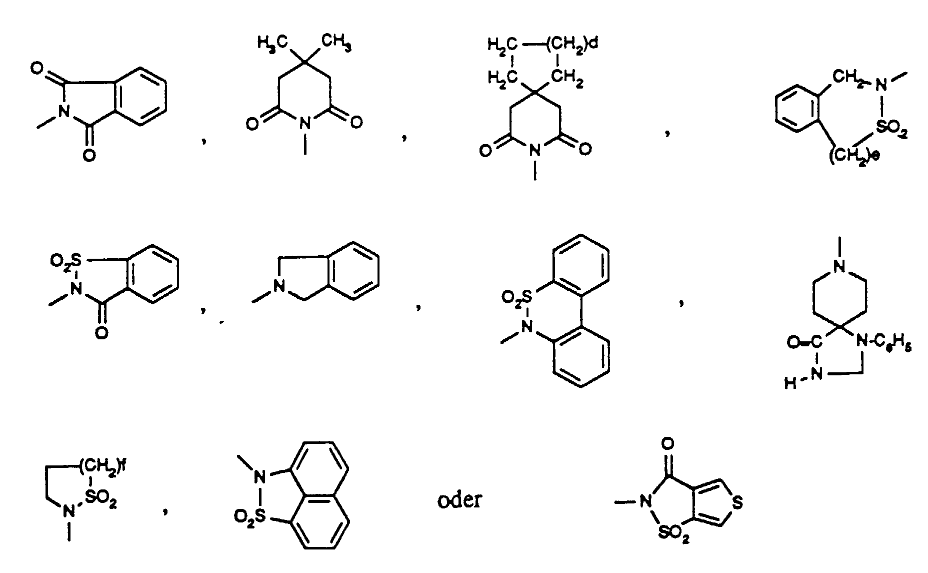 bilden,
worin
d und f gleich oder verschieden sind und die Zahl 1 oder 2 bedeuten,
e die Zahl 0, 1 oder 2 bedeutet,
R⁶ Wasserstoff, Cycloalkyl mit 3 bis 8 Kohlenstoffatomen, geradkettiges oder verzweigtes Alkyl, Alkylcarbonyl oder Carbamoyl mit jeweils bis zu 6 Kohlenstoffatomen bedeutet, oder
Benzyl, Aryl mit 6 bis 10 Kohlenstoffatomen oder einen 5-gliedrigen ungesättigten Heterocylus mit bis zu 4 Heteroatomen aus der Reihe N, S oder O bedeutet, die gegebenenfalls
durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
R⁷ Wasserstoff, geradkettiges oder verzweigtes Alkyl mit bis zu 6 Kohlenstoffatomen oder Benzyl bedeutet,
R⁸ und R⁹ gleich oder verschieden sind und Wasserstoff, geradkettiges oder verzweigtes Alkyl mit bis zu 8 Kohlenstoffatomen, Benzyl oder Aryl mit 6 bis 10 Kohlenstoffatomen bedeuten,
R³ für Wasserstoff, geradkettiges oder verzweigtes Alkyl mit bis zu 8 Kohlenstoffatomen oder Benzyl steht, oder
für die Gruppe -A-D steht,
worin
A und D die oben angegebene Bedeutung haben,
und deren Salze.

2. Aminomethyl-substituierte 2,3-Dihydropyrano[2,3-b]pyridine nach Anspruch 1, wobei
R¹ und R² gleich oder verschieden sind und
für Wasserstoff, Fluor, Chlor, Brom, Nitro, Trifluormethyl, Hydroxy oder für geradkettiges oder verzweigtes Alkyl oder Alkoxy mit jeweils bis zu 6 Kohlenstoffatomen stehen
a für die Zahl 0 oder 1 steht,
A für geradkettiges oder verzweigtes Alkylen oder Alkenylen mit jeweils bis zu 6 Kohlenstoffatomen steht,
D für Cyclopropyl, Cyclopentyl, Cyclohexyl oder Phenyl steht, oder
für eine Gruppe der Formel -NR⁴R⁵, -OR⁶, -CO₂R⁷, -CO-NR⁸R⁹ steht,
worin
R⁴ und R⁵ gleich oder verschieden sind und Wasserstoff, Cyclopropyl, Cyclopentyl, Cyclohexyl, Benzyl, geradkettiges oder verzweigtes Alkyl mit bis zu 6 Kohlenstoffatomen oder eine Gruppe der Formel -CO-R¹⁰ oder -SO₂R¹¹ bedeuten,
worin
R¹⁰ und R¹¹ gleich oder verschieden sind und geradkettiges oder verzweigtes Alkyl mit bis zu 6 Kohlenstoffatomen bedeuten, oder Benzyl oder Phenyl bedeuten, die gegebenenfalls durch Nitro, Cyano, Trifluormethyl, Fluor, Chlor, Carboxy, Hydroxy oder durch geradkettiges oder verzweigtes Alkyl, Alkoxy oder Alkoxycarbonyl mit jeweils bis zu 4 Kohlenstoffatomen substituiert sind,
oder
R⁴ und R⁵ gemeinsam mit dem Stickstoffatom einen Rest der Formel 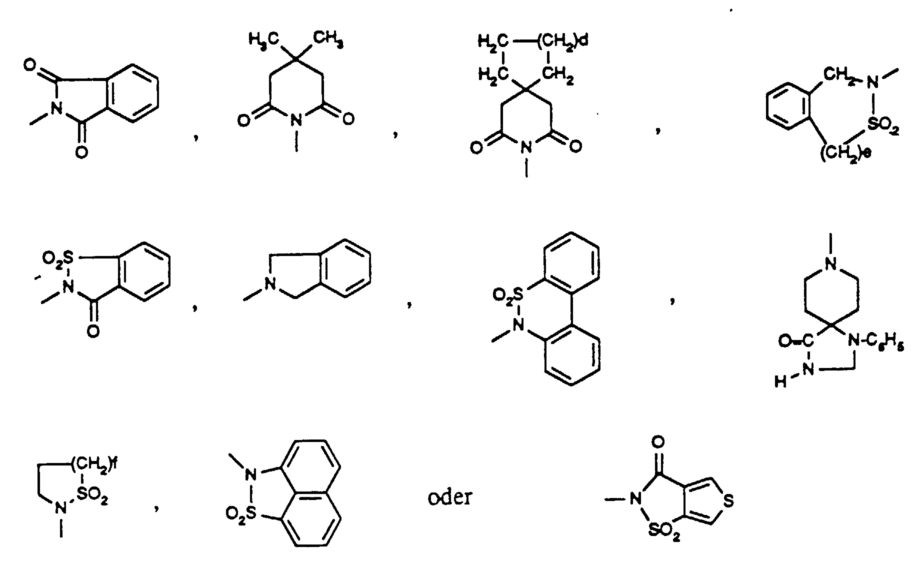 bilden,
worin
d und f gleich oder verschieden sind und die Zahl 1 oder 2 bedeuten,
e die Zahl 0, 1 oder 2 bedeutet,
R⁶ Wasserstoff, geradkettiges oder verzweigtes Alkyl, Alkylcarbonyl oder Carbamoyl mit jeweils bis zu 4 Kohlenstoffatomen bedeutet oder Benzyl oder Phenyl bedeutet, die gegebenenfalls durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
R⁷ Wasserstoff, geradkettiges oder verzweigtes Alkyl mit bis zu 4 Kohlenstoffatomen oder Benzyl bedeutet,
R⁸ und R⁹ gleich oder verschieden sind und Wasserstoff, geradkettiges oder verzweigtes Alkyl mit bis zu 6 Kohlenstoffatomen, Benzyl oder Phenyl bedeuten,
R³ für Wasserstoff, geradkettiges oder verzweigtes Alkyl mit bis zu 6 Kohlenstoffatomen oder Benzyl steht,
oder
für die Gruppe -A-D steht,
worin
A und D die oben angegebene Bedeutung haben,
und deren Salze.

3. Aminomethyl-substituierte 2,3-Dihydropyrano[2,3-b]pyridine nach Anspruch 1, wobei
R¹ und R² gleich oder verschieden sind und
für Wasserstoff, Fluor, Chlor, Trifluormethyl, Hydroxy oder für geradkettiges oder verzweigtes Alkyl oder Alkoxy mit jeweils bis zu 4 Kohlenstoffatomen stehen,
a für die Zahl 0 oder 1 steht,
A für geradkettiges oder verzweigtes Alkylen oder Alkenylen mit jeweils bis zu 4 Kohlenstoffatomen steht,
D für Cyclopropyl, Cyclopentyl, Cyclohexyl, Hydroxy oder Phenyl steht, oder
für eine Gruppe der Formel -NR⁴R⁵ steht,
worin
R⁴ und R⁵ gleich oder verschieden sind und Wasserstoff, Cyclopropyl, Cyclopentyl, Cyclohexyl, Benzyl oder geradkettiges oder verzweigtes Alkyl mit bis zu 4 Kohlenstoffatomen bedeuten,
oder
R⁴ und R⁵ gemeinsam mit dem Stickstoffatom einen Rest der Formel 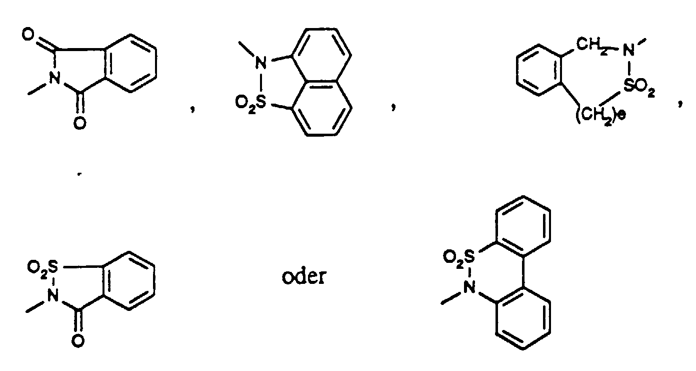 bilden,
worin
e die Zahl 0 oder 1 bedeutet,
R³ für Wasserstoff, geradkettiges oder verzweigtes Alkyl mit bis zu 4 Kohlenstoffatomen oder Benzyl steht,
oder
für die Gruppe -A-D steht,
worin
A und D die oben angegebene Bedeutung haben,
und deren Salze.

4. Verfahren zur Herstellung von Aminomethyl-substituierten 2,3-Dihydropyrano[2,3-b]pyridinen der allgemeinen Formel 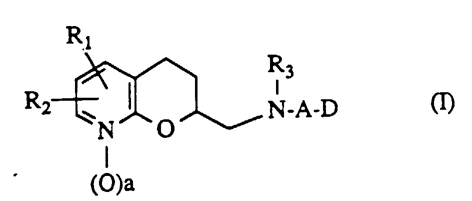 in welcher
R¹ und R² gleich oder verschieden sind und für Wasserstoff, Halogen, Nitro, Trifluormethyl, Hydroxy oder für geradkettiges oder verzweigtes Alkyl oder Alkoxy mit jeweils bis zu 8 Kohlenstoffatomen stehen,
a für die Zahl 0 oder 1 steht,
A für geradkettiges oder verzweigtes Alkylen oder Alkenylen mit jeweils bis zu 8 Kohlenstoffatomen steht,
D für Cycloalkyl mit 3 bis 8 Kohlenstoffatomen oder für Aryl mit 6 bis 10 Kohlenstoffatomen steht, oder
für eine Gruppe der Formel -NR⁴R⁵, -OR⁶, -CO₂R⁷, -CO-NR⁸R⁹ steht,
worin
R⁴ und R⁵ gleich oder verschieden sind und Wasserstoff, Cycloalkyl mit 3 bis 8 Kohlenstoffatomen, Benzyl, geradkettiges oder verzweigtes Alkyl mit bis zu 8 Kohlenstoffatomen oder eine Gruppe der Formel -CO-R¹⁰ oder -SO₂R¹¹ bedeuten,
worin
R¹⁰ und R¹¹ gleich oder verschieden sind und geradkettiges oder verzweigtes Alkyl mit bis zu 8 Kohlenstoffatomen bedeuten oder Benzyl, Aryl mit 6 bis 10 Kohlenstoffatomen oder einen 5- bis 7-gliedrigen ungesättigten Heterocyclus mit bis zu 4 Heteroatomen aus der Reihe S, N oder O bedeuten, die gegebenenfalls durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
oder
R⁴ und R⁵ gemeinsam mit dem Stickstoffatom einen Rest der Formel 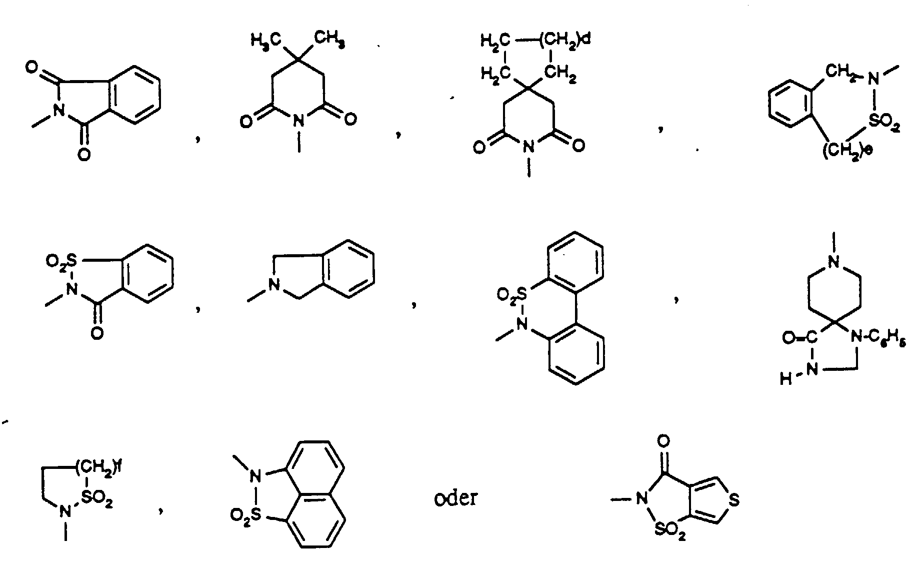 bilden,
worin
d und f gleich oder verschieden sind und die Zahl 1 oder 2 bedeuten,
e die Zahl 0, 1 oder 2 bedeutet,
R⁶ Wasserstoff, Cycloalkyl mit 3 bis 8 Kohlenstoffatomen, geradkettiges oder verzweigtes Alkyl, Alkylcarbonyl oder Carbamoyl mit jeweils bis zu 6 Kohlenstoffatomen bedeutet, oder
Benzyl, Aryl mit 6 bis 10 Kohlenstoffatomen oder einen 5-gliedrigen ungesättigten Heterocylus mit bis zu 4 Heteroatomen aus der Reihe N, S oder O bedeutet, die gegebenenfalls
durch Nitro, Cyano, Trifluormethyl, Halogen, Amino, Carboxy, Hydroxy oder durch geradkettiges oder verzweiges Alkyl, Alkoxy oder Alkoxycarbonyl mit jeweils bis zu 6 Kohlenstoffatomen substituiert sind,
R⁷ Wasserstoff, geadkettiges oder verzweigtes Alkyl mit bis zu 6 Kohlenstoffatomen oder Benzyl bedeutet,
R⁸ und R⁹ gleich oder verschieden sind und Wasserstoff, geradkettiges oder verzweigtes Alkyl mit bis zu 8 Kohlenstoffatomen, Benzyl oder Aryl mit 6 bis 10 Kohlenstoffatomen bedeuten,
R³ für Wasserstoff, geradkettiges oder verzweigtes Alkyl mit bis zu 8 Kohlenstoffatomen oder Benzyl steht, oder
für die Gruppe -A-D steht,
worin
A und D die oben angegebene Bedeutung haben,
und deren Salze,
dadurch gekennzeichnet, daß man 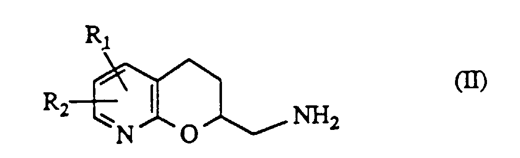 in welcher
R¹ und R² die oben angegebene Bedeutung haben,
mit Verbindungen der allgemeinen Formel (III)
L-A-D (III)
in welcher
A und D die oben angegebene Bedeutung haben
und
L für eine typische Abgangsgruppe, wie beispielsweise Brom, Chlor, Jod, Tosyl oder Mesyl, vorzugsweise für Brom steht,
in inerten Lösemitteln, gegebenenfalls in Anwesenheit einer Base und eines Reaktionsbeschleunigers umsetzt,
und im Fall, daß R³ nicht für Wasserstoff steht, eine Alkylierung,
und im Fall der 2,3-Dihydropyrano[2,3-b]pyridin-N-oxide -N-oxide (a = 1) eine Oxidation nach üblichen Methoden anschließt.

5. Arzneimittel enthaltend Aminomethyl-substituierte 2,3-Dihydropyrano[2,3-b]pyridine nach Anspruch 1.

6. Verfahren zur Herstellung von Arzneimitteln nach Anspruch 5, dadurch gekennzeichnet, daß die Aminomethyl-substituierten 2,3-Dihydropyrano[2,3-b]pyridine gegebenenfalls mit Hilfe von Hilfs- und Trägerstoffen in eine geeignete Applikationsform überführt werden.

7. Verwendung von Aminomethyl-substituierten 2,3-Dihydropyrano[2,3-b]pyridinen nach Anspruch 1 zur Herstellung von Arzneimitteln.

8. Amine der allgemeinen Formel 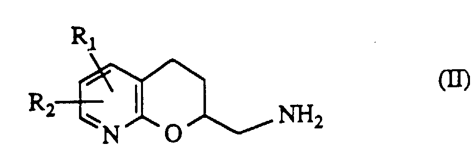 in welcher
R¹ und R² gleich oder verschieden sind und
- für Wasserstoff, Halogen, Nitro, Trifluormethyl, Hydroxy oder
- für geradkettiges oder verzweigtes Alkyl oder Alkoxy mit jeweils bis zu 8 Kohlenstoffatomen stehen.

9. Verfahren zur Herstellung der allgemeinen Formel 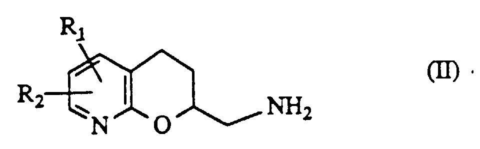 in welcher
R¹ und R² gleich oder verschieden sind und
- für Wasserstoff, Halogen, Nitro, Trifluormethyl, Hydroxy oder
- für geradkettiges oder verzweigtes Alkyl oder Alkoxy mit jeweils bis zu 8 Kohlenstoffatomen stehen,
dadurch gekennzeichnet, daß man Verbindungen der allgemeinen Formel 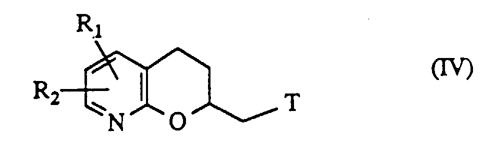 in welcher
R¹ und R² die oben angegebene Bedeutung haben,
und
T für Halogen, vorzugsweise für Brom und/oder Chlor steht,
gegebenenfalls als Gemisch (T-Br, T-Cl) zunächst mit Kaliumphthalimid in inerten Lösemitteln und unter Schutzgasatmosphäre zu den Verbindungen der allgemeinen Formel 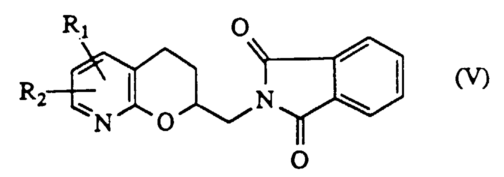 in welcher
R¹ und R² die oben angegebene Bedeutung haben,
umsetzt,
und in einem zweiten Schritt durch Umsetzung mit 2-Amino-ethanol oder Hydrazinhydrat die Aminfunktion freisetzt.

## Claims

1. Aminomethyl-substituted 2,3-dihydropyrano[2,3-b]pyridines of the general formula (I) 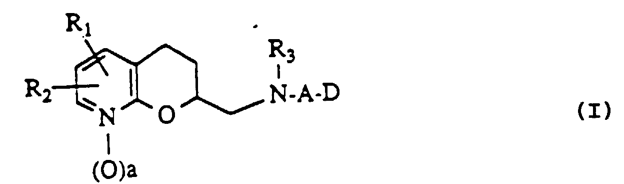 in which
R¹ and R² are identical or different and
represent hydrogen, halogen, nitro, trifluoromethyl, hydroxyl or straight-chain or branched alkyl or alkoxy each having up to 8 carbon atoms,
a represents the number 0 or 1,
A represents straight-chain or branched alkylene or alkenylene each having up to 8 carbon atoms,
D represents cycloalkyl having 3 to 8 carbon atoms or aryl having 6 to 10 carbon atoms, or represents a group of the formula -NR⁴R⁵, -OR⁶, -CO₂R⁷ or -CO-NR⁸R⁹,
in which
R⁴ and R⁵ are identical or different and denote hydrogen, cycloalkyl having 3 to 8 carbon atoms, benzyl, straight-chain or branched alkyl having up to 8 carbon atoms or a group of the formula -CO-R¹⁰ or -SO₂R¹¹,
in which
R¹⁰ and R¹¹ are identical or different and denote straight-chain or branched alkyl having up to 8 carbon atoms or benzyl, aryl having 6 to 10 carbon atoms or a 5- to 7-membered unsaturated heterocycle having up to 4 hetero atoms selected from the group comprising S, N or O, each of which is optionally substituted by nitro, cyano, trifluoromethyl, halogen, amino, carboxyl, hydroxyl or by straight-chain or branched alkyl, alkoxy or alkoxycarbonyl each having up to 6 carbon atoms,
or
R⁴ and R⁵ together with the nitrogen atom form a radical of the formula 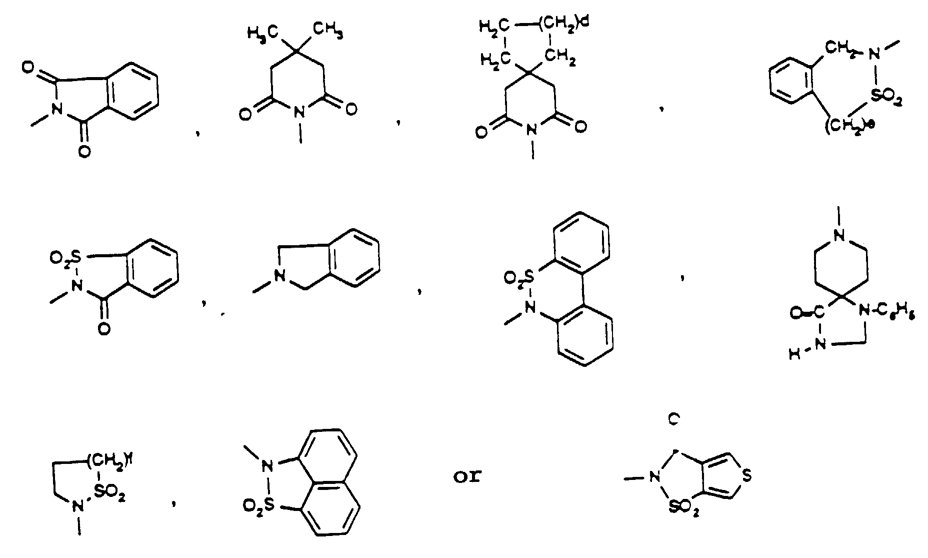 in which
d and f are identical or different and denote the number 1 or 2,
e denotes the number 0, 1 or 2,
R⁶ denotes hydrogen, cycloalkyl having 3 to 8 carbon atoms, straight-chain or branched alkyl, alkylcarbonyl or carbamoyl each having up to 6 carbon atoms, or
denotes benzyl, aryl having 6 to 10 carbon atoms or a 5-membered unsaturated heterocycle having up to 4 hetero atoms selected from the group comprising N, S or O, each of which is optionally substituted
by nitro, cyano, trifluoromethyl, halogen, amino, carboxyl, hydroxyl or by straight-chain or branched alkyl, alkoxy or alkoxycarbonyl each having up to 6 carbon atoms,
R⁷ denotes hydrogen, straight-chain or branched alkyl having up to 6 carbon atoms or benzyl,
R⁸ and R⁹ are identical or different and denote hydrogen, straight-chain or branched alkyl having up to 8 carbon atoms, benzyl or aryl having 6 to 10 carbon atoms,
R³ represents hydrogen, straight-chain or branched alkyl having up to 8 carbon atoms or benzyl, or represents the -A-D group,
in which
A and D have the meanings given above,
and their salts.

2. Aminomethyl-substituted 2,3-dihydropyrano[2,3-b]pyridines according to Claim 1, where
R¹ and R² are identical or different and
represent hydrogen, fluorine, chlorine, bromine, nitro, trifluoromethyl, hydroxyl or straight-chain or branched alkyl or alkoxy each having up to 6 carbon atoms,
a represents the number 0 or 1,
A represents straight-chain or branched alkylene or alkenylene each having up to 6 carbon atoms,
D represents cyclopropyl, cyclopentyl, cyclohexyl or phenyl, or represents a group of the formula -NR⁴R⁵, -OR⁶, -CO₂R⁷ or -CO-NR⁸R⁹,
in which
R⁴ and R⁵ are identical or different and denote hydrogen, cyclopropyl, cyclopentyl, cyclohexyl, benzyl, straight-chain or branched alkyl having up to 6 carbon atoms or a group of the formula -CO-R¹⁰ or -SO₂R¹¹,
in which
R¹⁰ and R¹¹ are identical or different and denote straight-chain or branched alkyl having up to 6 carbon atoms, or denote benzyl or phenyl, each of which is optionally substituted by nitro, cyano, trifluoromethyl, fluorine, chlorine, carboxyl, hydroxyl or by straight-chain or branched alkyl, alkoxy or alkoxycarbonyl each having up to 4 carbon atoms,
or
R⁴ and R⁵ together with the nitrogen atom form a radical of the formula 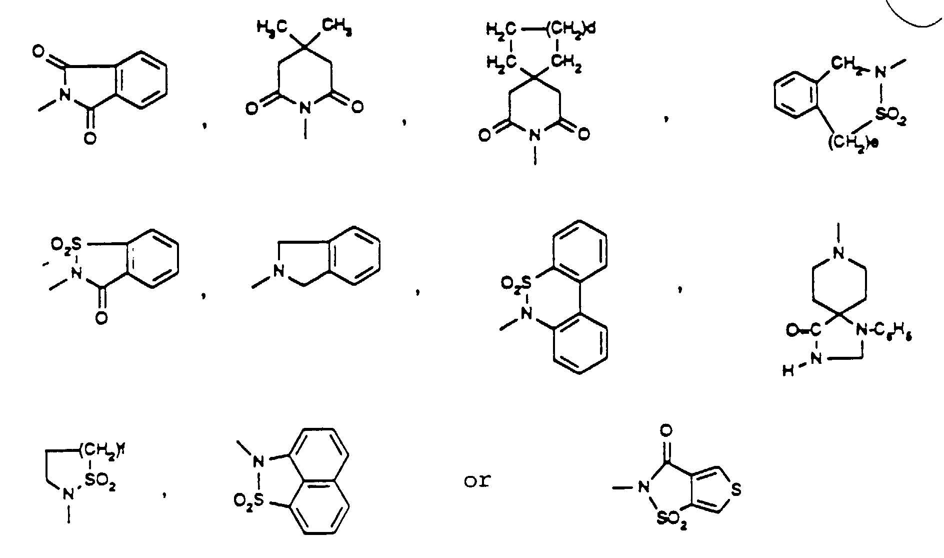 in which
d and f are identical or different and denote the number 1 or 2,
e denotes the number 0, 1 or 2,
R⁶ denotes hydrogen, straight-chain or branched alkyl, alkylcarbonyl or carbamoyl each having up to 4 carbon atoms,
or denotes benzyl or phenyl, each of which is optionally substituted by nitro, cyano, trifluoromethyl, halogen, amino, carboxyl, hydroxyl or by straight-chain or branched alkyl, alkoxy or alkoxycarbonyl each having up to 6 carbon atoms,
R⁷ denotes hydrogen, straight-chain or branched alkyl having up to 4 carbon atoms or benzyl,
R⁸ and R⁹ are identical or different and denote hydrogen, straight-chain or branched alkyl having up to 6 carbon atoms, benzyl or phenyl,
R³ represents hydrogen, straight-chain or branched alkyl having up to 6 carbon atoms or benzyl,
or
represents the -A-D group,
in which
A and D have the meanings given above,
and their salts.

3. Aminomethyl-substituted 2,3-dihydropyrano[2,3-b]pyridines according to Claim 1, where
R¹ and R² are identical or different and
represent hydrogen, fluorine, chlorine, trifluoromethyl, hydroxyl or straight-chain or branched alkyl or alkoxy each having up to 4 carbon atoms,
a represents the number 0 or 1,
A represents straight-chain or branched alkylene or alkenylene each having up to 4 carbon atoms,
D represents cyclopropyl, cyclopentyl, cyclohexyl, hydroxyl or phenyl, or represents a group of the formula -NR⁴R⁵,
in which
R⁴ and R⁵ are identical or different and denote hydrogen, cyclopropyl, cyclopentyl, cyclohexyl, benzyl or straight-chain or branched alkyl having up to 4 carbon atoms,
or
R⁴ and R⁵ together with the nitrogen atom form a radical of the formula 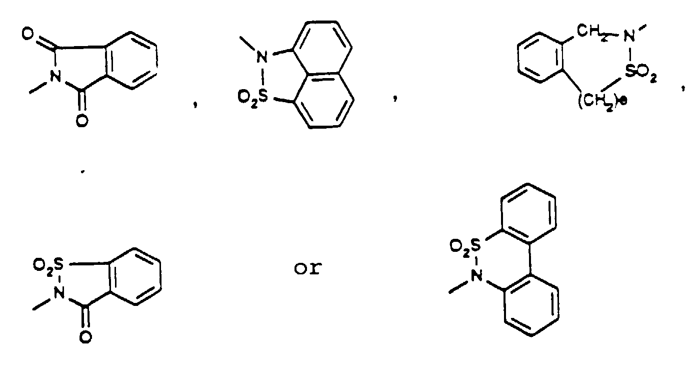 in which
e denotes the number 0 or 1,
R³ represents hydrogen, straight-chain or branched alkyl having up to 4 carbon atoms or benzyl,
or
represents the -A-D group,
in which
A and D have the meanings given above,
and their salts.

4. Process for the preparation of aminomethyl-substituted 2,3-dihydropyrano[2,3-b]pyridines of the general formula (I) 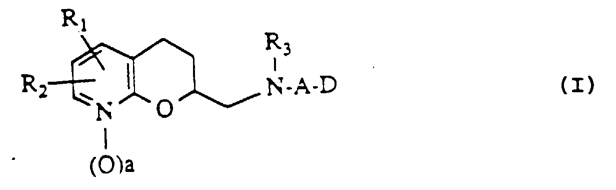 in which
R¹ and R² are identical or different and
represent hydrogen, halogen, nitro, trifluoromethyl, hydroxyl or straight-chain or branched alkyl or alkoxy each having up to 8 carbon atoms,
a represents the number 0 or 1,
A represents straight-chain or branched alkylene or alkenylene each having up to 8 carbon atoms,
D represents cycloalkyl having 3 to 8 carbon atoms or aryl having 6 to 10 carbon atoms, or represents a group of the formula -NR⁴R⁵, -OR⁶, -CO₂R⁷ or -CO-NR⁸R⁹,
in which
R⁴ and R⁵ are identical or different and denote hydrogen, cycloalkyl having 3 to 8 carbon atoms, benzyl, straight-chain or branched alkyl having up to 8 carbon atoms or a group of the formula -CO-R¹⁰ or -SO₂R¹¹,
in which
R¹⁰ and R¹¹ are identical or different and denote straight-chain or branched alkyl having up to 8 carbon atoms or denote benzyl, aryl having 6 to 10 carbon atoms or a 5- to 7-membered unsaturated heterocycle having up to 4 hetero atoms selected from the group comprising S, N or O, each of which is optionally substituted by nitro, cyano, trifluoromethyl, halogen, amino, carboxyl, hydroxyl or by straight-chain or branched alkyl, alkoxy or alkoxycarbonyl each having up to 6 carbon atoms,
or
R⁴ and R⁵ together with the nitrogen atom form a radical of the formula 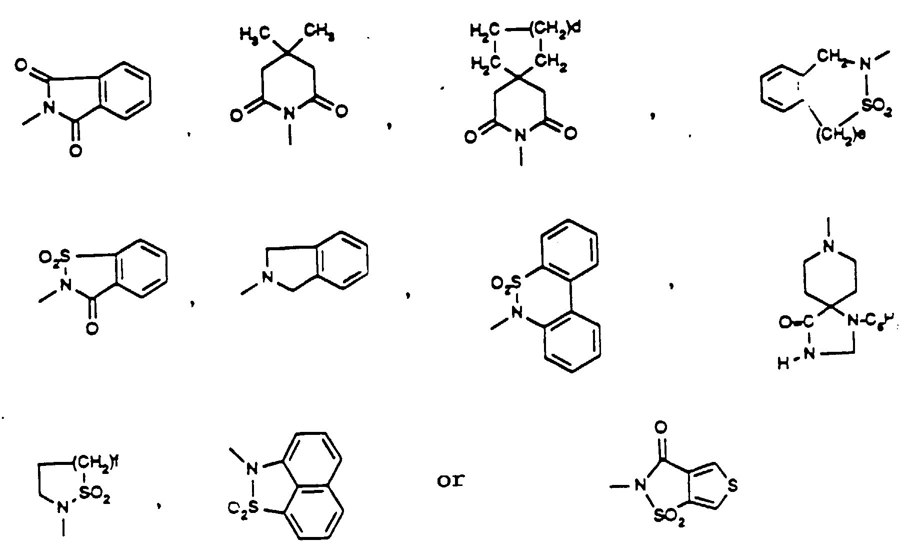 in which
d and f are identical or different and denote the number 1 or 2,
e denotes the number 0, 1 or 2,
R⁶ denotes hydrogen, cycloalkyl having 3 to 8 carbon atoms, straight-chain or branched alkyl, alkylcarbonyl or carbamoyl each having up to 6 carbon atoms, or
denotes benzyl, aryl having 6 to 10 carbon atoms or a 5-membered unsaturated heterocycle having up to 4 hetero atoms selected from the group comprising N, S or O, each of which is optionally substituted
by nitro, cyano, trifluoromethyl, halogen, amino, carboxyl, hydroxyl or by straight-chain or branched alkyl, alkoxy or alkoxycarbonyl each having up to 6 carbon atoms,
R⁷ denotes hydrogen, straight-chain or branched alkyl having up to 6 carbon atoms or benzyl,
R⁸ and R⁹ are identical or different and denote hydrogen, straight-chain or branched alkyl having up to 8 carbon atoms, benzyl or aryl having 6 to 10 carbon atoms,
R³ represents hydrogen, straight-chain or branched alkyl having up to 8 carbon atoms or benzyl, or
represents the -A-D group,
in which
A and D have the meanings given above,
and their salts,
characterised in that
amines of the general formula (II) 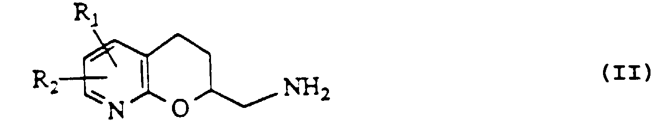 in which
R¹ and R² have the meanings given above,
are reacted with compounds of the general formula (III)
L-A-D (III)
in which
A and D have the meanings given above
and
L represents a typical leaving group, such as for example bromine, chlorine, iodine, tosyl or mesyl, preferably bromine,
in inert solvents, optionally in the presence of a base and a reaction accelerator,
and for the case that R³ does not represent hydrogen, there is a subsequent alkylation,
and for the case of the 2,3-dihydropyrano[2,3-b]pyridine N-oxides (a=1), there is a subsequent oxidation, in each case according to conventional methods.

5. Medicaments containing aminomethyl-substituted 2,3-dihydropyrano[2,3-b]pyridines according to Claim 1.

6. Process for the preparation of medicaments according to Claim 5, characterised in that the aminomethyl-substituted 2,3-dihydropyrano [2,3-b] pyridines are converted into a suitable administration form, optionally with the aid of adjuncts and excipients.

7. Use of aminomethyl-substituted 2,3-dihydropyrano[2,3-b]pyridines according to Claim 1 for the preparation of medicaments.

8. Amines of the general formula 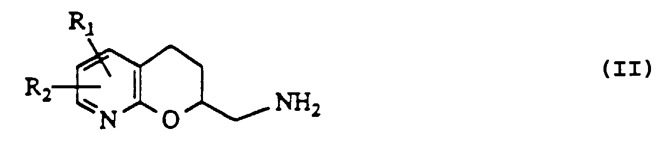 in which
R¹ and R² are identical or different and
- represent hydrogen, halogen, nitro, trifluoromethyl, hydroxyl or
- straight-chain or branched alkyl or alkoxy each having up to 8 carbon atoms.

9. Process for the preparation of the general formula 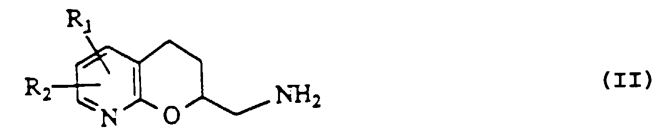 in which
R¹ and R² are identical or different and
- represent hydrogen, halogen, nitro, trifluoromethyl, hydroxyl or
- straight-chain or branched alkyl or alkoxy each having up to 8 carbon atoms,
characterised in that compounds of the general formula 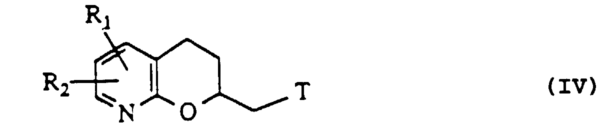 in which
R¹ and R² have the meanings given above,
and
T represents halogen, preferably bromine and/or chlorine,
are reacted optionally as a mixture (T-Br, T-Cl), firstly with potassium phthalimide in inert solvents and under a protective gas atmosphere to give compounds of the general formula 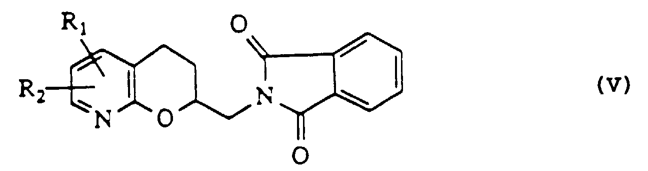 in which
R¹ and R² have the meanings given above,
and in a second step, the amine function is liberated by reaction with 2-amino-ethanol or hydrazine hydrate.

## Revendications

1. 2,3-dihydropyranno[2,3-b]pyridines à substituant aminométhyle, de formule générale 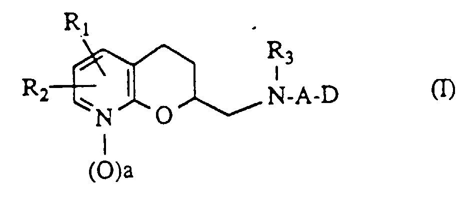 dans laquelle
R¹ et R² sont identiques ou différents et représentent de l'hydrogène, un halogène, un groupe nitro, trifluorométhyle, hydroxy, ou un groupe alkyle ou alkoxy linéaire ou ramifié ayant chacun jusqu'à 8 atomes de carbone,
a représente le nombre 0 ou 1,
A est un groupe alkylène ou alcénylène linéaire ou ramifié ayant chacun jusqu'à 8 atomes de carbone,
D est un groupe cycloalkyle ayant 3 à 8 atomes de carbone ou un groupe aryle ayant 6 à 10 atomes de carbone, ou bien
un groupe de formule -NR⁴R⁵, -OR⁶, -CO₂R⁷, -CO-NR⁸R⁹, dans laquelle
R⁴ et R⁵ sont identiques ou différents et représentent de l'hydrogène, un groupe cycloalkyle ayant 3 à 8 atomes de carbone, un groupe benzyle, un groupe alkyle linéaire ou ramifié ayant jusqu'à 8 atomes de carbone ou un groupe de formule -CO-R¹⁰ ou -SO₂R¹¹,
dans laquelle
R¹⁰ et R¹¹ sont identiques ou différents et représentent un groupe alkyle linéaire ou ramifié ayant jusqu'à 8 atomes de carbone ou un groupe benzyle, un groupe aryle ayant 6 à 10 atomes de carbone ou un hétérocycle non saturé pentagonal à heptagonal ayant jusqu'à 4 hétéroatomes de la série S, N ou O, qui sont substitués, le cas échéant, par un groupe nitro, cyano, trifluorométhyle, un halogène, un groupe amino, carboxy, hydroxy ou par un groupe alkyle, alkoxy ou alkoxycarbonyle linéaire ou ramifié ayant chacun jusqu'à 6 atomes de carbone,
ou bien
R⁴ et R⁵ forment, conjointement avec l'atome d'azote, un reste de formule 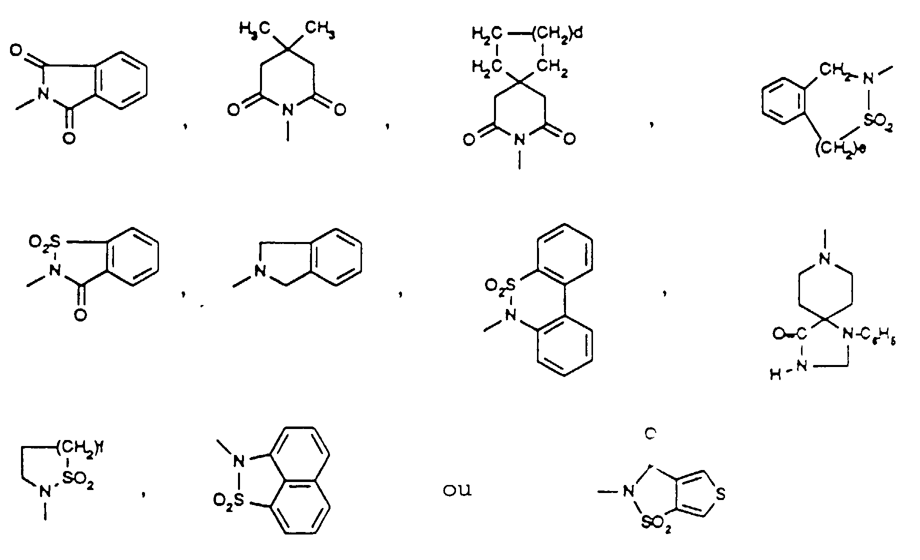 dans laquelle
d et f sont identiques ou différents et représentent le nombre 1 ou 2,
e est le nombre 0, 1 ou 2,
R⁶ est de l'hydrogène, un groupe cycloalkyle ayant 3 à 8 atomes de carbone, un groupe alkyle, alkylcarbonyle ou carbamoyle linéaire ou ramifié ayant chacun jusqu'à 6 atomes de carbone, ou bien
un groupe benzyle, aryle ayant 6 à 10 atomes de carbone ou un hétérocycle pentagonal non saturé ayant jusqu'à 4 hétéroatomes de la série N, S ou O, qui sont substitués, le cas échéant, par un groupe nitro, cyano, trifluorométhyle, un halogène, un groupe amino, carboxy, hydroxy ou par un groupe alkyle, alkoxy ou alkoxycarbonyle linéaire ou ramifié ayant chacun jusqu'à 6 atomes de carbone,
R⁷ est de l'hydrogène, un groupe alkyle linéaire ou ramifié ayant jusqu'à 6 atomes de carbone ou un groupe benzyle,
R⁸ et R⁹ sont identiques ou différents et représentent de l'hydrogène, un groupe alkyle linéaire ou ramifié ayant jusqu'à 8 atomes de carbone, un groupe benzyle ou un groupe aryle ayant 6 à 10 atomes de carbone,
R³ est de l'hydrogène, un groupe alkyle linéaire ou ramifié ayant jusqu'à 8 atomes de carbone ou un groupe benzyle, ou bien
le groupe -A-D,
dans lequel
A et D ont la définition indiquée ci-dessus,
et leurs sels.

2. 2,3-dihydropyranno[2,3-b]pyridines à substituant aminométhyle suivant la revendication 1, dans lesquelles
R¹ et R² sont identiques ou différents et représentent de l'hydrogène, du fluor, du chlore, du brome, un groupe nitro, trifluorométhyle, hydroxy ou un groupe alkyle ou alkoxy linéaire ou ramifié ayant chacun jusqu'à 6 atomes de carbone,
a représente le nombre 0 ou 1,
A est un groupe alkylène ou un groupe alcénylène linéaire ou ramifié ayant chacun jusqu'à 6 atomes de carbone,
D est un groupe cyclopropyle, cyclopentyle, cyclohexyle ou phényle,
ou bien un groupe de formule -NR⁴R⁵, -OR⁶, -CO₂R⁷, -CO-NR⁸R⁹, dans laquelle
R⁴ et R⁵ sont identiques ou différents et représentent de l'hydrogène, un groupe cyclopropyle, cyclopentyle, cyclohexyle, benzyle, un groupe alkyle linéaire ou ramifié ayant jusqu'à 6 atomes de carbone ou un groupe de formule -CO-R¹⁰ ou -SO₂R¹¹,
dans laquelle
R¹⁰ et R¹¹ sont identiques ou différents et représentent un groupe alkyle linéaire ou ramifié ayant jusqu'à 6 atomes de carbone, ou un groupe benzyle ou un groupe phényle, qui sont substitués, le cas échéant, par un groupe nitro, cyano, trifluorométhyle, du fluor, du chlore, un groupe carboxy, hydroxy ou par un groupe alkyle, alkoxy ou alkoxycarbonyle linéaire ou ramifié ayant chacun jusqu'à 4 atomes de carbone,
ou bien
R⁴ et R⁵ forment, conjointement avec l'atome d'azote, un reste de formule 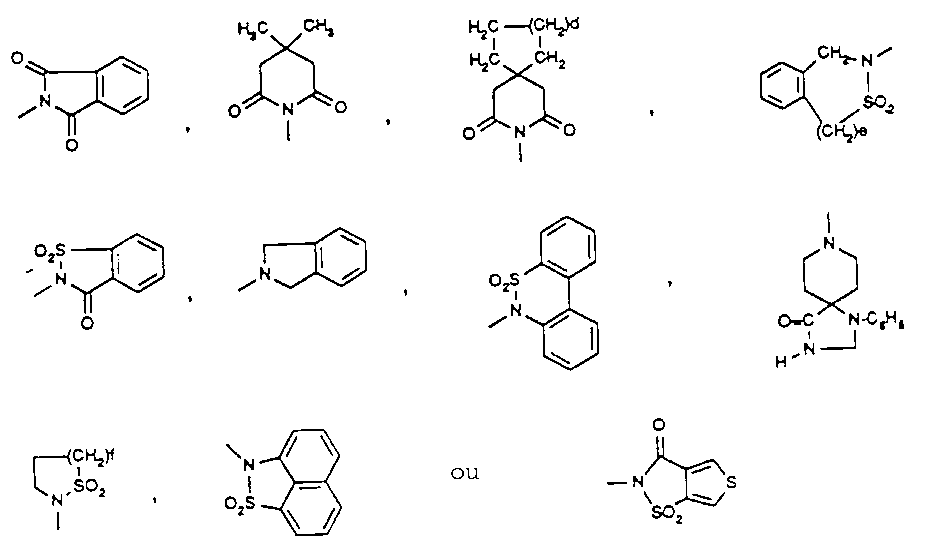 dans laquelle
d et f sont identiques ou différents et représentent le nombre 1 ou 2,
e est le nombre 0, 1 ou 2,
R⁶ est de l'hydrogène, un groupe alkyle, alkylcarbonyle ou un groupe carbamoyle linéaire ou ramifié ayant chacun jusqu'à 4 atomes de carbone, ou bien
un groupe benzyle ou un groupe phényle, qui sont substitués, le cas échéant, par un radical nitro, cyano, trifluorométhyle, halogéno, amino, carboxy, hydroxy ou par un radical alkyle, alkoxy ou alkoxycarbonyle linéaire ou ramifié ayant chacun jusqu'à 6 atomes de carbone,
R⁷ est de l'hydrogène, un groupe alkyle linéaire ou ramifié ayant jusqu'à 4 atomes de carbone ou un groupe benzyle,
R⁸ et R⁹ sont identiques ou différents et représentent de l'hydrogène, un groupe alkyle linéaire ou ramifié ayant jusqu'à 6 atomes de carbone, un groupe benzyle ou phényle,
R³ est de l'hydrogène, un groupe alkyle linéaire ou ramifié ayant jusqu'à 6 atomes de carbone ou un groupe benzyle, ou bien
le groupe -A-D,
dans lequel
A et D ont la définition indiquée ci-dessus,
et leurs sels.

3. 2,3-dihydropyranno[2,3-b]pyridines à substituant aminométhyle suivant la revendication 1, dans lesquelles
R¹ et R² sont identiques ou différents et représentent de l'hydrogène, du fluor, du chlore, un groupe trifluorométhyle, hydroxy, ou bien un groupe alkyle ou un groupe alkoxy linéaire ou ramifié ayant chacun jusqu'à 4 atomes de carbone,
a représente le nombre 0 ou 1,
A est un groupe alkylène ou un groupe alcénylène linéaire ou ramifié ayant chacun jusqu'à 4 atomes de carbone,
D est un groupe cyclopropyle, cyclopentyle, cyclohexyle, hydroxy ou phényle,
ou bien un groupe de formule -NR⁴R⁵,
dans laquelle
R⁴ et R⁵ sont identiques ou différents et représentent de l'hydrogène, un groupe cyclopropyle, cyclopentyle, cyclohexyle, benzyle, ou un groupe alkyle linéaire ou ramifié ayant jusqu'à 4 atomes de carbone,
ou bien
R⁴ et R⁵ forment, conjointement avec l'atome d'azote, un reste de formule 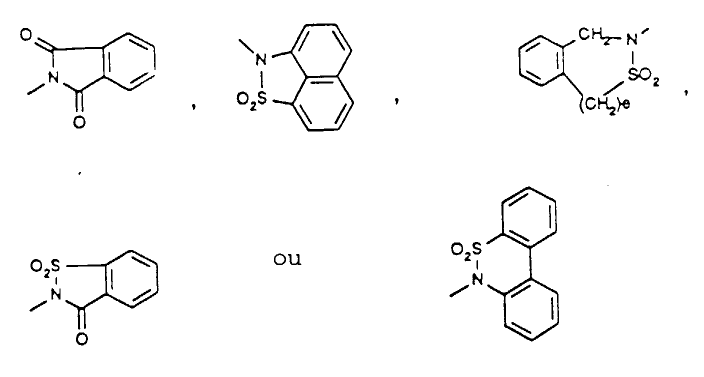 dans laquelle
e représente le nombre 0 ou 1,
R³ est de l'hydrogène, un groupe alkyle linéaire ou ramifié ayant jusqu'à 4 atomes de carbone ou un groupe benzyle,
ou bien
le groupe -A-D,
dans lequel
A et D ont la définition indiquée ci-dessus,
et leurs sels.

4. Procédé de production de 2,3-dihydropyranno[2,3-b]pyridines à substituant aminométhyle, de formule générale 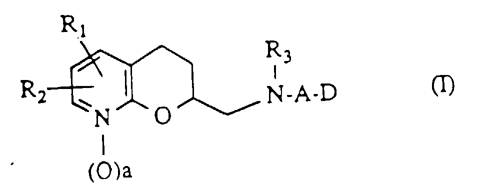 dans laquelle
R¹ et R² sont identiques ou différents et représentent de l'hydrogène, un halogène, un groupe nitro, trifluorométhyle, hydroxy, ou un groupe alkyle ou alkoxy linéaire ou ramifié ayant chacun jusqu'à 8 atomes de carbone,
a représente le nombre 0 ou 1,
A est un groupe alkylène ou alcénylène linéaire ou ramifié ayant chacun jusqu'à 8 atomes de carbone,
D est un groupe cycloalkyle ayant 3 à 8 atomes de carbone ou un groupe aryle ayant 6 à 10 atomes de carbone, ou bien
un groupe de formule -NR⁴R⁵, -OR⁶, -CO₂R⁷, -CO-NR⁸R⁹,
dans laquelle
R⁴ et R⁵ sont identiques ou différents et représentent de l'hydrogène, un groupe cycloalkyle ayant 3 à 8 atomes de carbone, un groupe benzyle, un groupe alkyle linéaire ou ramifié ayant jusqu'à 8 atomes de carbone ou un groupe de formule -CO-R¹⁰ ou -SO₂R¹¹,
dans laquelle
R¹⁰ et R¹¹ sont identiques ou différents et représentent un groupe alkyle linéaire ou ramifié ayant jusqu'à 8 atomes de carbone ou un groupe benzyle, un groupe aryle ayant 6 à 10 atomes de carbone ou un hétérocycle non saturé pentagonal à heptagonal ayant jusqu'à 4 hétéroatomes de la série S, N ou O, qui sont substitués, le cas échéant, par un groupe nitro, cyano, trifluorométhyle, un halogène, un groupe amino, carboxy, hydroxy ou par un groupe alkyle, alkoxy ou alkoxycarbonyle linéaire ou ramifié ayant chacun jusqu'à 6 atomes de carbone,
ou bien
R⁴ et R⁵ forment, conjointement avec l'atome d'azote, un reste de formule 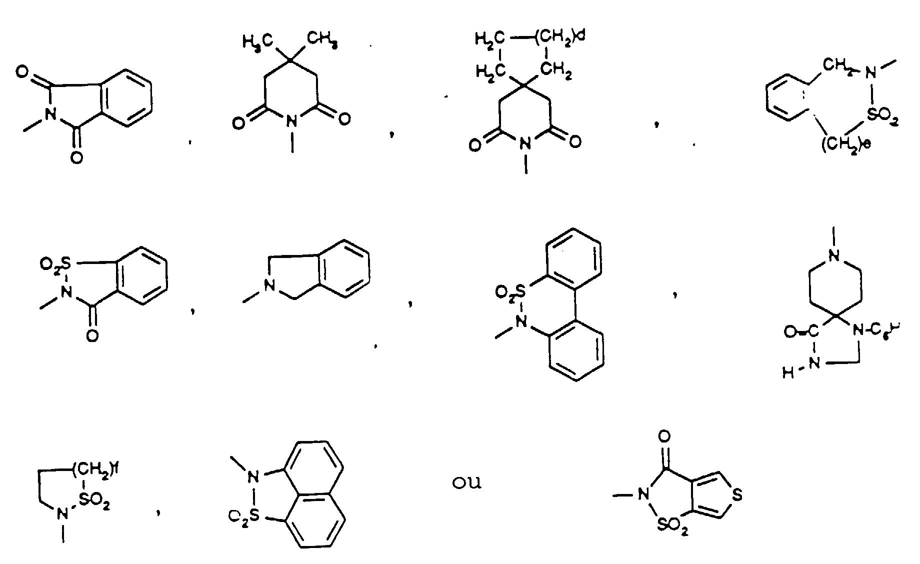 dans laquelle
d et f sont identiques ou différents et représentent le nombre 1 ou 2,
e est le nombre 0, 1 ou 2,
R⁶ est de l'hydrogène, un groupe cycloalkyle ayant 3 à 8 atomes de carbone, un groupe alkyle, alkylcarbonyle ou carbamoyle linéaire ou ramifié ayant chacun jusqu'à 6 atomes de carbone, ou bien
un groupe benzyle, aryle ayant 6 à 10 atomes de carbone ou un hétérocycle pentagonal non saturé ayant jusqu'à 4 hétéroatomes de la série N, S ou O, qui sont substitués, le cas échéant, par un groupe nitro, cyano, trifluorométhyle, un halogène, un groupe amino, carboxy, hydroxy ou par un groupe alkyle, alkoxy ou alkoxycarbonyle linéaire ou ramifié ayant chacun jusqu'à 6 atomes de carbone,
R⁷ est de l'hydrogène, un groupe alkyle linéaire ou ramifié ayant jusqu'à 6 atomes de carbone ou un groupe benzyle,
R⁸ et R⁹ sont identiques ou différents et représentent de l'hydrogène, un groupe alkyle linéaire ou ramifié ayant jusqu'à 8 atomes de carbone, un groupe benzyle ou un groupe aryle ayant 6 à 10 atomes de carbone,
R³ est de l'hydrogène, un groupe alkyle linéaire ou ramifié ayant jusqu'à 8 atomes de carbone ou un groupe benzyle, ou bien
le groupe -A-D,
dans lequel
A et D ont la définition indiquée ci-dessus,
et leurs sels,
caractérisé en ce qu'on fait réagir des amines de formule générale (II) 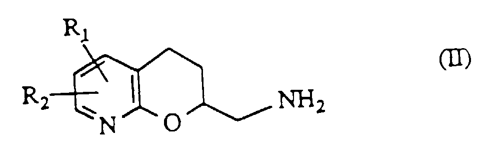 dans laquelle
R¹ et R² ont la définition indiquée ci-dessus,
avec des composés de formule générale (III)
L-A-D (III)
dans laquelle
A et D ont la définition indiquée ci-dessus,
et
L est un groupe partant typique tel que, par exemple, du brome, du chlore, de l'iode, un groupe tosyle ou mésyle, de préférence du brome,
dans des solvants inertes, éventuellement en présence d'une base et d'un accélérateur de réaction,
et au cas où R³ ne représente pas de l'hydrogène, on effectue ensuite une alkylation,
et dans le cas des N-oxydes de 2,3-dihydropyranno[2,3-b]pyridines (a = 1), on effectue ensuite une oxydation, par des procédés classiques.

5. Médicaments contenant des 2,3-dihydropyranno[2,3-b)pyridines à substituant aminométhyle suivant la revendication 1.

6. Procédé de préparation de médicaments suivant la revendication 5, caractérisé en ce qu'on fait prendre aux 2,3-dihydropyranno[2,3-b]pyridines à substituant aminométhyle une forme qui convient pour l'administration, éventuellement à l'aide de substances auxiliaires et de supports.

7. Utilisation de 2,3-dihydropyranno[2,3-b)pyridines à substituant aminométhyle suivant la revendication 1 pour la préparation de médicaments.

8. Amines de formule générale 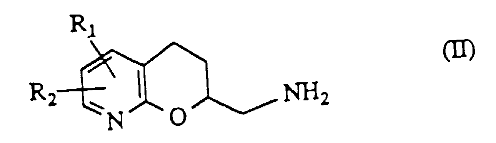 dans laquelle
R¹ et R² sont identiques ou différents et représentent
- de l'hydrogène, un halogène, un groupe nitro, trifluorométhyle, hydroxy,
ou bien
- un groupe alkyle ou un groupe alkoxy linéaire ou ramifié ayant chacun jusqu'à 8 atomes de carbone.

9. Procédé de production des amines de formule générale 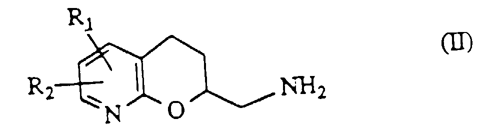 dans laquelle
R¹ et R² sont identiques ou différents et représentent
- de l'hydrogène, un halogène, un groupe nitro, trifluorométhyle, hydroxy
ou bien
- un groupe alkyle ou un groupe alkoxy linéaire ou ramifié ayant chacun jusqu'à 8 atomes de carbone,
caractérisé en ce qu'on fait réagir des composés de formule générale 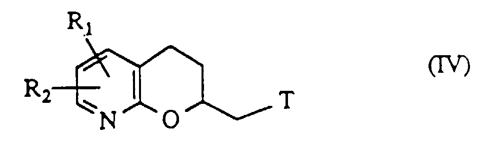 dans laquelle
R¹ et R² ont la définition indiquée ci-dessus
et
T représente un halogène, de préférence le brome et/ou le chlore,
le cas échéant, sous forme de mélange (T-Br, T-Cl), tout d'abord avec le phtalimidure de potassium dans des solvants inertes et sous atmosphère de gaz protecteur pour obtenir les composés de formule générale 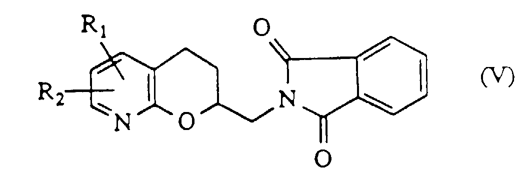 dans laquelle
R¹ et R² ont la définition indiquée ci-dessus,
et dans une seconde étape, on libère la fonction amino par réaction avec le 2-amino-éthanol ou l'hydrate d'hydrazine.
